# EUROPEAN PATENT SPECIFICATION

(11) **EP 4 590 349 B1**
(45) Date of publication and mention of the grant of the patent: **28.10.2026**
(21) Application number: 23783020.3
(22) Date of filing: 06.09.2023
(51) Int. Cl.: A61L 27/10, A61L 27/56, A61L 27/16, A61L 27/12, A61L 27/58

(54) **SOLID AND COMPACT BIOMATERIAL, METHOD FOR ITS PREPARATION AND KIT FOR FILLING BONE CAVITIES**
FESTES UND KOMPAKTES BIOMATERIAL, VERFAHREN ZU SEINER HERSTELLUNG UND KIT ZUM FÜLLEN VON KNOCHENHOHLRÄUMEN
BIOMATÉRIAU SOLIDE ET COMPACT, SON PROCÉDÉ DE PRÉPARATION ET KIT DE REMPLISSAGE DE CAVITÉS OSSEUSES

(30) Priority: 23.09.2022 IT 202200019566
(43) Date of publication of application: 30.07.2025
(73) Proprietor: TECRES S.P.A., 37066 Sommacampagna (VR) (IT)
(72) Inventor: FACCIOLI, Giovanni, 37066 Sommacampagna (Verona) (IT); SOFFIATTI, Renzo, 37066 Sommacampagna (Verona) (IT)
(74) Representative: Feltrinelli, Secondo Andrea
(86) International application number: PCT/IB2023/058796
(87) International publication number: WO 2024/062316

(56) References cited:
- US-A1- 2013 274 891
- BERUTO D T ET AL: "USE OF ALPHA-TRICALCIUM PHOSPHATE (TCP) AS POWDERS AND AS AN AQUEOUS DISPERSION TO MODIFY PROCESSING, MICROSTRUCTURE, AND MECHANICAL PROPERTIES OF POLYMETHYLMETHACRYLATE (PMMA) BONE CEMENTS AND TO PRODUCE BONE-SUBSTITUTE COMPOUNDS", JOURNAL OF BIOMEDICAL MATERIALS RESEARCH, WILEY, NEW YORK, NY, US, vol. 49, 1 January 2000 (2000-01-01), pages 498 - 505, XP001070173, ISSN: 0021-9304, DOI: 10.1002/(SICI)1097-4636(20000315)49:4<498::AID-JBM8>3.0.CO;2-1
- PIRCHER NICOLE ET AL: "Preparation and Reinforcement of Dual-Porous Biocompatible Cellulose Scaffolds for Tissue Engineering", vol. 300, no. 9, 28 April 2015 (2015-04-28), DE, pages 911 - 924, XP093043432, ISSN: 1438-7492, Retrieved from the Internet <URL:https://onlinelibrary.wiley.com/doi/full-xml/10.1002/mame.201500048> [retrieved on 20230502], DOI: 10.1002/mame.201500048

## Description

### TECHNICAL FIELD OF THE INVENTION

The present invention is defined by the appended claims and concerns a solid and compact biomaterial that can be used for filling bone gaps, even large ones.

The present invention also refers to a method for the production of such biomaterial as well as to a system or kit made up of such biomaterial in solid form and in fluid form for filling bone gaps.

The present invention also relates to a bone substitute comprising a biomaterial according to the present invention.

This biomaterial is able to integrate optimally with the biological system in which it interfaces, therefore presenting improved characteristics in terms of bioinductivity, bioconductivity and mechanical performance.

### TECHNICAL BACKGROUND

In the biomaterials sector, in particular biomaterials that can be used in the orthopedic and surgical fields, to fill cavities or gaps of various origins and types, ceramic and/or polymeric biomaterials, mostly, are currently used, which are substantially used to fix prostheses or as bone substitutes.

Among these polymeric biomaterials there are, for example, acrylic bone cement, possibly composed of polymethyl methacrylate (PMMA) and/or methyl methacrylate (MMA), and reabsorbable cements, the latter used in cases where the support function they must perform is limited in time.

PMMA is the plastic resin present in acrylic bone cement, a two-component medical product composed of a powder and a liquid kept separate until used by the doctor. The powder is made up of approximately 85% PMMA polymer (methyl methacrylate polymer) and the liquid is made up of 98% MMA (methyl methacrylate monomer). Other components such as radiopacifying agents, catalysts and antibiotics may be present in small quantities. Once mixed together, the PMMA powder and the MMA liquid give rise to the formation of a malleable and injectable fluid mass which acquires and increases viscosity over time and solidifies within about 10 minutes by heating to around 70-80°C. The reaction that occurred is the polymerization reaction of the MMA monomer which in turn becomes a polymer or PMMA. The solid obtained is in fact PMMA containing the agents present at the origin and also a portion (around 5-10%) of unreacted MMA. In this way, bone cement is formed, usually used to support metal joint prostheses, for example in patients' legs, for many years (even more than 20 years if desired). It follows that the more mechanically robust the bone cement is, the longer it lasts over time and the fewer structural failures which unfortunately rarely occur. In this regard, there are standards, such as ISO5833 and ASTM F 451-21, specific for PMMA which define the minimum mechanical resistance to compression that the product must possess.

However, an important disadvantage of acrylic bone cement is given by the fact that it does not integrate with the bone tissue surrounding the implant site, rather it determines the formation of a fibrous sheath, or a mantle of fibrous tissue, at the interface between the biomaterial itself and the bone tissue, with consequent risk of laxity in the implant.

As regards, however, reabsorbable cements, they are more biocompatible than acrylic cement but have poor mechanical resistance.

There are also biomaterials that have micro-cavities and macro-cavities of certain dimensions to facilitate bone regrowth within them. However, these materials have mechanical characteristics that are valid for some applications but not for others. Furthermore, they may present internal inhomogeneities, once polymerized, which affect their capillary capacity.

Known bone substitutes can also comprise human bone tissue, suitably deantigenated, or a ceramic material.

However, the former can undergo early reabsorption or mechanical failure, leaving dangerous empty spaces in the site where they were previously implanted, while the latter do not guarantee, in most cases, adequate structural support but are dissoluble in the human body. Furthermore, they can become infected, are very expensive and difficult to produce and can give rise to immune rejection.

Known ceramic materials are mainly composed of mono-ceramic materials (such as for example hydroxyapatite) or bi-ceramic materials (such as for example hydroxyapatite + tricalcium phosphate) or poly-ceramic materials (such as for example mixtures of different ceramics or different calcium salts, of the type hydroxyapatite + tricalcium phosphate + calcium sulphate).

The use of one type or another of ceramic material depends on the speed of dissolution of the material itself once implanted in the human body: if very slow or almost zero reabsorption is desired, hydroxyapatite will be used, while calcium sulphate guarantees on the contrary a very rapid reabsorption. Ceramic mixtures with different solubilities will exploit this aspect according to therapeutic needs.

However, ceramic materials are very fragile and can break easily, releasing dangerous fragments. For this reason, like human bone products, they must always be coupled with metal supports (e.g. plates, fixators, etc.). Even during dissolution, they produce fragments that can migrate and cause serious inflammatory reactions.

Hybrid materials for implantation in the human body are also known comprising water-soluble plastic polymers, for example formed by polylactic and polyglycolic acid, alone or in a mixture of the same or racemic forms of the two, in association with TCP and HA. This type of material, however, once implanted is destined to disappear completely over time as all its constituents are completely soluble.

This type of material has not given very positive results and is mostly used to make absorbable screws and not bone substitutes.

A document disclosing bone substitutes is US 2013/274891 A1, which discloses a bone filling material comprising a porous PMMA structural component having an interconnected porosity and is prepared by a process which comprises providing the PMMA in powder form and a ceramic component, in particular TCP, in powder and granular form.

There is therefore a need to have a biomaterial available to be used as a bone substitute, perfectly capable of integrating with the bone tissue surrounding the implant site, to the point of allowing blood circulation to be recreated within it, and at the same time having properties improved mechanics even in the case, for example, of large lesions or bone gaps.

### OBJECTS OF THE INVENTION

The technical task of the present invention is therefore to improve the state of the art.

Within the scope of this technical task, it is an aim of the present invention to develop a solid and compact biomaterial with improved mechanical properties.

Another object of the present invention is to develop a biomaterial with improved capillary properties.

In accordance with one aspect of the present invention, a biomaterial according to claim 1 is provided.

A further object of the present invention is to develop a method for the production of a solid and compact biomaterial which is simple and practical.

In accordance with one aspect of the present invention, a method according to claim 10 is provided.

A still further object of the present invention is to develop a system or kit comprising a solid and compact biomaterial as well as a fluid biomaterial for filling bone gaps.

In accordance with one aspect of the present invention, a system or kit according to claim 15 is provided.

In accordance with one aspect of the present invention, a bone substitute according to claim 18 is provided.

The dependent claims refer to preferred and advantageous forms of the invention.

### BRIEF DESCRIPTION OF THE DRAWINGS

These and further advantages will be better understood by any person skilled in the art from the following description and the attached drawings, given as a nonlimiting example, in which:
figure 1 illustrates some examples of biomaterial conformations according to the present invention;
figure 2 illustrates some further examples of biomaterial conformations according to the present invention;
figure 3 illustrates an example of implantation of the biomaterial according to the present invention (comprising acrylic resin and/or PMMA, in combination with TCP) in an animal;
figure 4 illustrates four specimens (n°1-n°4) subjected to tests for performance analysis in terms of mechanical properties;
figure 5 illustrates a detail of specimen no. 3 of figure 4;
figure 6 illustrates the mechanical characteristics compared to specimen no. 1 (on the left) and specimen no. 3 (on the right) of figure 4;
figure 7 illustrates a graph in which the test time is indicated on the abscissa (in seconds) while the load (in Newton) is indicated on the ordinate for the four specimens n°1-n°4 of figure 4,
figures 8A and 8B illustrate an enlarged detail of a version of the biomaterial according to the present invention formed respectively by PMMA and β-TCP and PMMA alone, as the β-TCP has been dissolved and/or reabsorbed,
figure 9 illustrates a graph relating to an example of distribution of the granules chosen with a nominal grain size of 200-500 microns, in which the size in microns is on the abscissa while the percentage quantity is on the ordinate,
figures 10A and 10B illustrate a version of the biomaterial according to the present invention formed respectively by PMMA and β-TCP before implantation and PMMA alone, as the β-TCP has been dissolved and/or reabsorbed,
figure 11 illustrates an example of a cranial prosthesis that can be created with the biomaterial according to a version of the present invention, for example a custom-made cranial prosthesis,
figure 12 illustrates an example of a spacer device, for example a shoulder device, which can be made with the biomaterial according to a version of the present invention,
figure 13 illustrates three specimens made with the same resin (first component, for example PMMA) polymerized under vacuum (A), for example at a pressure of -0.8 bar, at an ambient pressure of 1 bar (B) and at a pressure of 10 bar (C), for example in an autoclave,
figure 14 illustrates the section of the specimens of figure 13, in which this section has been rubbed with vaseline, for example stringy, (top row of figure 14), and with carbon black (bottom row of figure 14).

### EMBODIMENTS OF THE INVENTION.

In this specification, biomaterial means a material capable of interfacing with a biological system for the purpose of increasing, treating or replacing any tissue, organ or function of the body; bioinductivity means the ability of the biomaterial to induce the neoformation of the tissue with which this biomaterial interfaces; bioconductivity means the ability of the biomaterial to create a suitable support, capable of allowing the colonization of the progenitor cells of the tissue with which this material interfaces and capable of guaranteeing their survival and proliferation.

The biomaterial according to the present invention has biocompatibility properties, is bioinductive and/or bioconductive.

The biomaterial according to the present invention is suitable for filling gaps or bony cavities, for example of large dimensions, such as for example large lesions caused by war wounds, large tumors, large traumas, for example automotive, etc.

However, it is understood that the peculiarities of the present biomaterial allow it to be used to fill bone lesions or gaps in general, for example for those areas of the human body that are subjected to high or greater loads than other areas.

The biomaterial according to the present invention is therefore suitable for use as a bone substitute in bone gaps or cavities or even in functional restorations (of tissues) in which the insertion, replacement or restoration of bone tissue is necessary in use.

The biomaterial according to the present invention is in the form of a preformed solid and preferably has a three-dimensional spherical, hemispherical, discoid or discoidal, polyhedral, parallelepiped, hexahedron, truncated cone or truncated pyramid, cone or pyramid, parallelepiped, rod or splint, chock-shaped, wedge-shaped, or an irregular three-dimensional shape. This shape and/or conformation is the one it presents when ready for use, for example in use at the time of implantation in the human body.

The biomaterial according to the present invention, therefore, being solid and preformed, is supplied in this state to the surgeon for his use. It is, in fact, a product prepared in a factory and not by a doctor.

In fact, therefore, it is a product that can be supplied to the surgeon as it is, and/or that can be inserted as it is or after milling to better adapt it to specific shapes and/or dimensions, in the respective bone cavities to make up for the lack of bone and/or in specific implant seats.

Being solid and preformed, the biomaterial according to the present invention also constitutes - in at least one version - a bone substitute. Therefore, unless specifically indicated otherwise, the properties described for the biomaterial can also be understood as belonging to the bone substitute itself.

By bone substitute we mean a preformed solid capable of being used for filling lesions, gaps or bone cavities or for the functional restoration of tissues in which the replacement or restoration of bone tissue is necessary in use, for example for large gaps or bones cavities, large lesions caused by war wounds, large tumors, large traumas, for example caused by car accidents, etc.

By bone substitute we can also mean a prosthetic or spacer device 100, permanent or temporary, to be implanted in the human body in bone seats or gaps, for the replacement of parts of bone tissue or joints damaged and/or compromised by pathologies or infections.

The biomaterial according to the present invention, therefore, can constitute a cranial prosthesis 100, for the reconstruction of cranial damage, or even a preformed spacer device 100 for the removal of infected prostheses and for the consequent treatment of the infection present in the bone cavity infects thanks to its structure and porosity which make it capable, for example, of absorbing and then releasing in vivo a medical substance such as a specific antibiotic.

With the biomaterial of the present invention, solid and preformed, small devices of a few grams can be produced up to devices of 100 grams or more if necessary.

When in the present specification the bone substitute is mentioned, it is meant - as better defined below - made by a first structural component of permanent and in use not resorbable in the human body, wherein the first structural component comprises an organic, plastic and/or polymer material, and an interconnected porosity comprising pores and/or canaliculi having diameter less than 100 microns, micro-cavities having dimensions less than 100 microns and macro-cavities having dimensions between 100 and 500 microns or between 200 microns and 500 microns or between 300 and 500 microns or between 100 microns and 2000 microns or between 200 microns and 2000 microns or between 300 microns and 2000 microns or greater than 250 microns and up to 2000 microns or greater than 250 microns and up to 500 microns or between 500 microns and 2000 microns or greater than 500 microns and up to 2000 microns. In the bone substitute according to the present invention, which is defined by the appended claims, these micro-cavities and macro-cavities can be formed during the polymerization and/or solidification step of the first structural component and become hollow following the dissolution and/or solubilization of a second ceramic component, housed in at least some of the same. Otherwise, these micro-cavities and macro-cavities can be made differently, depending on the needs.

Furthermore, the second component are housed in the micro-cavities and macro-cavities. This means, especially as regards the macro-cavities, that they can be at least only partially occupied by the second component.

Before the dissolution of the second component, therefore, the biomaterial according to the present invention presents such micro-cavities and macro-cavities but they are not hollow (or not all hollow), as the second component is housed in at least some of the same, preferably in all micro-cavities and macro-cavities. In this phase, therefore, the cavities act as a housing seat for the second component.

The dissolution of the second component can occur before implantation or after implantation, depending on specific needs.

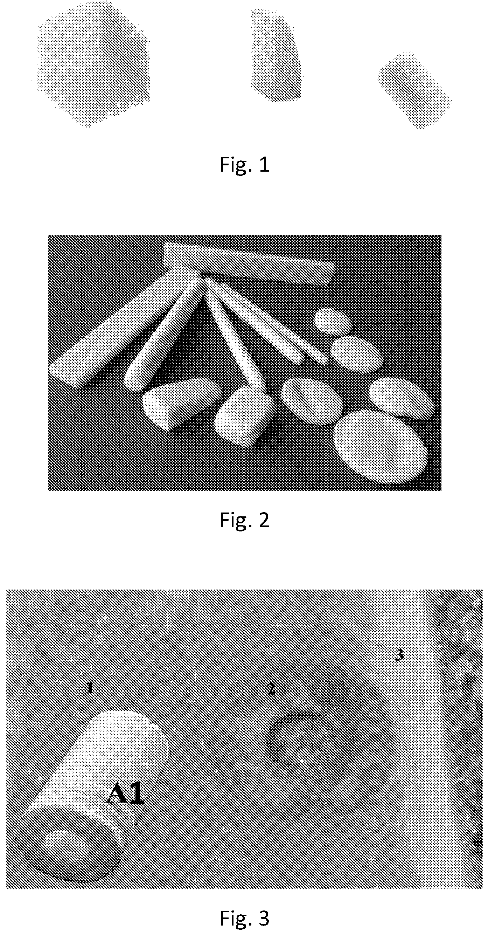

Examples of possible conformations of the biomaterial according to the present invention are illustrated by way of example in figures 1 and 2.

This biomaterial therefore forms a solid artefact. By artefact we mean a product derived from hand or machine manufacturing.

This biomaterial is therefore in solid form and, as will be better specified below, compact.

As will be understood from the present disclosure, the biomaterial according to the present invention is, before implantation and at least for a certain period after its implantation, a composite and/or mixed biomaterial in terms of the materials that compose it.

The biomaterial according to the present invention, in fact, includes a first structural component and a second ceramic component.

This second ceramic component is soluble, for example in use in contact with fluids, for example biological.

The first structural component is permanent and forms a structural matrix that characterizes the biomaterial according to the present invention. This means that, once implanted in the human body, it is not reabsorbed nor does it dissolve or erode due to the effect of fluids, for example biological or otherwise. The first structural component is therefore insoluble and keeps the original shape unchanged.

The first structural component and/or the structural matrix are shaped like a "sponge".

The first structural component, in fact, is porous. **In** particular, the biomaterial includes "open" pores, meaning they allow the passage of liquids by capillarity. This allows, together with other characteristics highlighted below, to preserve the biological activity of the surrounding tissue and to promote the regrowth of the bone tissue inside it too.

The pores are interconnected and arranged in a random but homogeneous way throughout the whole volume occupied by this first structural component.

This porosity can include a plurality of "canaliculi", for example with a diameter of less than 100 microns, which are formed during the manufacturing step of the biomaterial itself.

The canaliculi are also communicating and interconnected with each other and with the remaining porosity of the biomaterial according to the present invention.

The first structural component, therefore, is both compact and porous.

The first structural component comprises or is made up of at least one plastic and/or polymeric component. This material is naturally biocompatible. In one version of the invention, the first structural component comprises a plastic material, for example a plastic or acrylic or methacrylic resin, or a plastic or acrylic polymer or a bone cement made of polymethyl methacrylate (PMMA) and/or a copolymer based on methyl methacrylate or a thermoplastic polymer, polyethylene, polypropylene, polyester, a thermoformable polymer or other similar materials.

The presence of the aforementioned "canaliculi" is an essential requirement so that the various materials that constitute the first structural component can promote bone regrowth within them. The material of this first structural component, in fact, as mentioned, has a conformation and/or a state of matter that corresponds to a "sponge", i.e. for example completely similar to spongy bone tissue. The characteristic of being spongy is given precisely to the presence of porosity and/or canaliculi inside the first structural component.

The second ceramic material, in fact, absolutely could not be reached - at least the one present in the depth of the first structural component - by fluids, for example water or biological fluids, for its dissolution, if the first structural component was not porous and/or did not present canaliculi.

Following this, the dissolution of the second ceramic component, with the consequent formation of empty spaces within the first structural component, allows a positive biological response to be triggered, which guarantees bone regrowth within the biomaterial.

In one version of the invention, the first structural component consists (only) of at least one of the aforementioned materials.

The first structural component is made of an organic material.

In at least one version of the present invention, the first structural component is not a polysaccharide and/or the biomaterial according to the present invention does not comprise a polysaccharide.

The first structural component includes micro-cavities and macro-cavities.

These micro-cavities and macro-cavities are hollow in use, at least after a certain period of time after implantation.

These micro-cavities and macro-cavities can therefore also be part of the porosity of the biomaterial.

Such micro-cavities and macro-cavities can be made hollow, in at least one version of the present invention, by the empty space left by the dissolution of the second ceramic component in the first structural component.

The micro-cavities have a size of less than 100 microns.

The macro-cavities preferentially include a size or diameter between 200 microns and 500 microns or between 300 microns and 500 microns and, in gradually decreasing number or percentage, even larger diameters up to 2000 microns or between 100 and 500 microns or between 100 and 2000 microns or between 200 microns and 2000 microns or between 300 microns and 2000 microns or above 250 microns up to 2000 microns or above 500 microns up to 2000 microns. The macro-cavities present a substantially spheroidal conformation. The macro-cavities are the result of the presence of the second component, for example of a granule of the same, incorporated into the structural component, from the dissolution of which the micro-cavity is hollow and/or free.

The micro-cavities and macro-cavities are homogeneously distributed throughout the whole volume constituted by the biomaterial and/or by the first structural component of the biomaterial according to the present invention.

The canaliculi are also homogeneously distributed throughout the whole volume made up of the biomaterial and/or the first structural component and with respect to the position of the micro-cavities and macro-cavities.

The second ceramic component is made of an inorganic material.

It is therefore understandable why the biomaterial according to the present invention is composite: it is a "hybrid" material made up of a plastic and/or polymeric material and a ceramic material.

The second ceramic component is in the form of a powder having a size of less than 100 microns and granules having a size of between 100 microns and 500 microns or between 100 microns and 2000 microns or between 200 microns and 500 microns or between 300 microns and 500 microns or between 200 microns to 2000 microns or between 300 microns and 2000 microns or greater than 250 microns up to 2000 microns or greater than 500 microns up to 2000 microns.

According to at least one version of the present invention, the granules have a grain size between 100 microns and 2000 microns being a statistical mixture of granules with different grain sizes.

For example, a typical distribution of the granules chosen with a nominal grain size of 200-500 microns is reported below:

**Table 1a: particle size distribution**

| Granulometry | Quantity % (by weight on the total weight of the second component) |
|---|---|
| < 100 microns | 5 |
| >100 microns and <200 microns | 35 |
| >200 microns and <500 microns | 45 |
| >500 microns and <1000 microns | 10 |
| >1000 microns and <2000 microns | 5 |

For example, a typical distribution of the granules chosen with a nominal grain size of 300-500 microns is reported below:

**Table 1b: particle size distribution**

| Granulometry | Quantity % (by weight on the total weight of the second component) |
|---|---|
| < 100 microns | 5 |
| >100 microns and <300 microns | 35 |
| >300 microns and <500 microns | 45 |
| >500 microns and <1000 microns | 10 |
| >1000 microns and <2000 microns | 5 |

The second ceramic component includes at least one calcium salt.

This calcium salt may comprise: a compound based on calcium phosphate (CP), a calcium sulphate (CS), calcium carbonate (CC), etc.

Among the calcium phosphate-based compounds, at least one or more of the following may be present: calcium dihydrogen phosphate monohydrate (MCPM), calcium dihydrogen phosphate anhydrous (MCPA), calcium monohydrogen phosphate dihydrate (DCPD), calcium monohydrogen phosphate anhydrous (DCP), octacalcium phosphate (OCP), alpha-tricalcium phosphate (α-TCP), beta-tricalcium phosphate (β-TCP), hydroxyapatite (HA) tetracalcium phosphate (TTCP).

In at least one version of the present invention, the second ceramic component comprises at least two or more calcium salts.

According to one version of the invention, the second ceramic component comprises only calcium phosphate or a mixture of calcium phosphate with calcium sulphate and/or calcium carbonate and/or hydroxyapatite.

For example, according to one version of the present invention, the second ceramic component comprises calcium sulphate and/or calcium carbonate and/or hydroxyapatite and/or tricalcium phosphate.

According to an example of the present invention, the second ceramic component can be composed of a mixture of hydroxyapatite and calcium carbonate, for example according to a ratio HA:CC = 30%:70%.

The presence of (at least) one calcium salt is very important because it enhances the bio-tolerance and integration of the biomaterial with the surrounding tissues in use.

For example, if the biomaterial is used inserted both partly in a bone tissue and partly in a medullary fat tissue, the interfaces of the biomaterial itself at these (different) tissues integrate with them by incorporating their progenitor cells such as osteoblasts, without fibrotic encapsulations typical of the hostile rejection response. These neo-tissue cells then progress into mature lamellar tissue, expanding into the empty spaces left by the dissolution of the second ceramic component, promoting true osteointegration.

The choice of different calcium salts is also linked to the difference in their dissolution/solution times. The different solubilization times of the calcium salts allow the doctor to adapt the biomaterial to the biological recovery times of the bone affected by the implant which are not the same for all types of bone and also depend on the mechanical loads acting on the implant site.

For example, the regeneration of the skull bone which is practically not subject to load proceeds at a different pace than that of the femur or tibia which, on the contrary, are subjected to interacting forces of many kilograms.

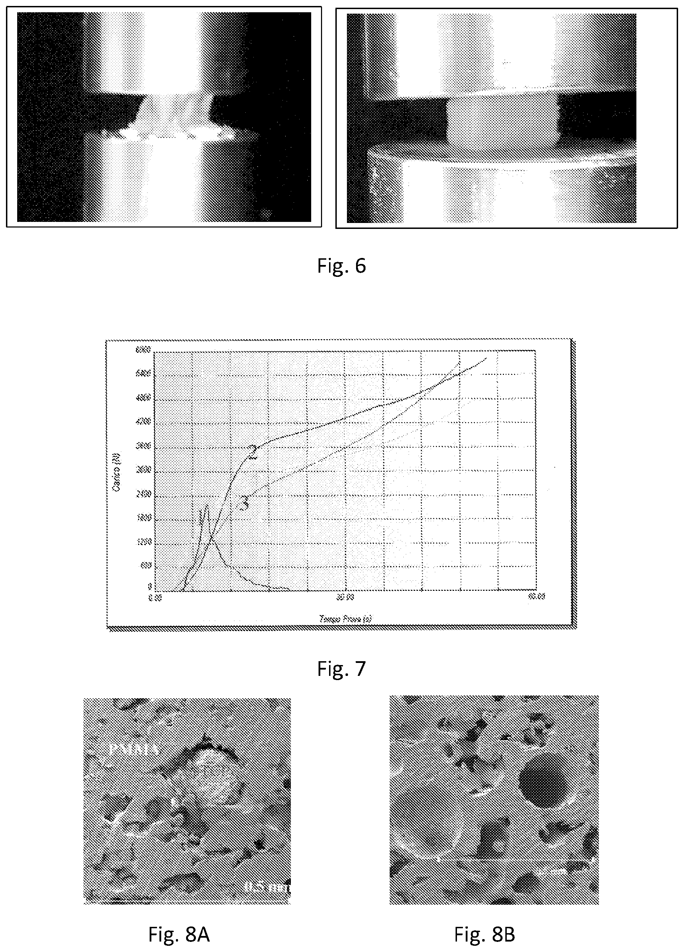

Considering figures 8A and 8B, an initial condition of the solid biomaterial is illustrated respectively (fig. 8A), in which TCP granules of the second ceramic component are embedded in the canaliculi and/or in general in the porosity of the first structural component, which in this case is PMMA. The "spongy" nature of PMMA is noted.

Figure 8B, however, shows the final situation of the biomaterial, i.e. after the dissolution of the TCP granules by the fluids, for example water, liquids or biological fluids, which, through the connected network of canaliculi, have reached and dissolved these granules. In these cavities, left empty by the dissolution of the second ceramic component, the bone tissue settles, as these cavities (called macro-cavities) have a size that allows the formation of mature lamellar bone.

We report in Table 2 a list of calcium phosphate-based compounds based on their dissolution speed. The tip of the arrow has a lower dissolution rate while the beginning of the arrow has a higher dissolution rate.

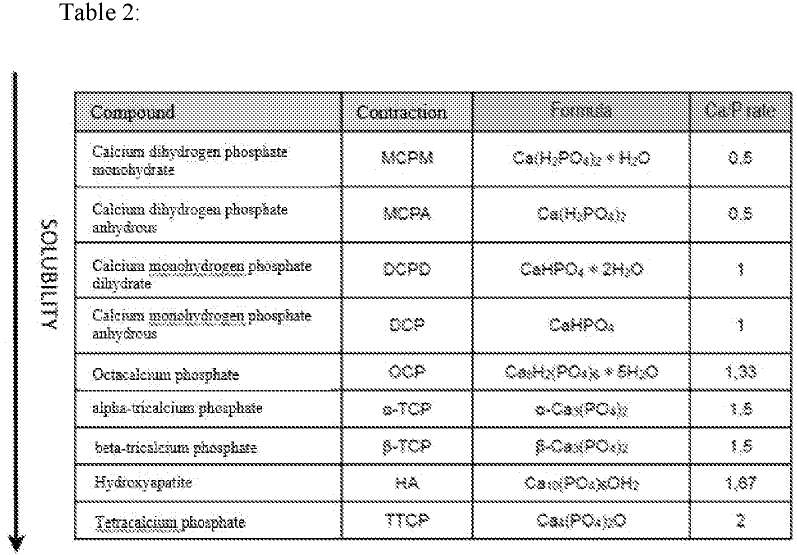

In Table 3 below we report some solubility values in water.

**Table 3:**

| **Calcium Salt** | **Solubility in water** |
|---|---|
| Calcium Sulphate (CaSO₄) | 2g/l |
| Calcium Carbonate (CaCO₃) | 0.014g/l |
| Tricalcium phosphate (Ca₃(PO₄)₂ TCP | 0.002g/l |
| Hydroxyapatite Ca₁₀(PO₄)₆(OH)₂ HA | Pratically insoluble |

The second ceramic component occupies the space present inside the micro-cavities and macro-cavities of the first structural component.

In particular, the granules of the second ceramic component occupy the macro-cavities while the powder, thanks to its size, occupies the micro-cavities (and possibly also the canaliculi).

Naturally this is an approximate location, as in fact it is the presence of the second component during the polymerization and/or solidification phase of the first component that determines the seats (i.e. at least part of the porosity and/or the micro-cavities and the macro-cavities) in which the second component itself is housed.

Subsequently, the dissolution of the powder creates the patency of the micro-cavities while the (subsequent) dissolution of the granules determines the patency of the macro-cavities.

The canaliculi are formed independently (and in advance) with respect to the patency of the micro-cavities and macro-cavities, and therefore to the formation of the complete porosity of the biomaterial of the present invention, as they are formed during the polymerization step of the first component, when substantially the dissolution of the second component has not yet occurred.

The first structural component that constitutes the biomaterial according to the present invention performs a structural function and has certain mechanical properties, much superior to known materials.

Furthermore, being insoluble, it remains permanently present in the implant (only the second ceramic component will be solubilized and/or reabsorbed, in any case it will be at least partially eliminated from the biomaterial and/or replaced following the implant). This condition gives the biomaterial according to the present invention mechanical performances that are almost constant over time, as they depend (at least for the most part and/or when the second ceramic component is completely dissolved) on the quantity and/or type of plastic material insoluble present. Therefore, the need for accessory mechanical support (plates, external fixator, etc.) is less compelling and even useless.

Furthermore, this biomaterial is not fragile and does not give rise to any fragmentation during the solubilization of the second ceramic component. In fact, the biomaterial in question is much more elastic than bone and even than prior art materials: its elastic modulus is, in fact, 0.8-3.0 GPa, based on the percentages and/or types of the second component ceramic. On the contrary, current materials, being ceramic in nature, are much more rigid and fragile. Hydroxyapatite, for example, has an elastic modulus that varies from 40 to 117 GPa, making it much stiffer than bone tissue whose modulus is around 18 GPa.

As mentioned, the second ceramic component is soluble and therefore, after coming into contact with water and/or liquids of various kinds and/or biological liquids, for example once the biomaterial has been implanted in use in the human body, it has the ability to dissolve and/or dissolve gradually.

For this reason, during use, a first period of time can be distinguished during which the biomaterial is implanted in the human body which is made up of the first structural component and the second ceramic component (the latter in percentage of 100%, that is, not yet dissolved, solubilized and/or reabsorbed). There is then a second period of time, following the first, during which the second ceramic component begins to dissolve and/or solubilise in contact with water or liquids, for example biological, and a third and final period of time in which the biomaterial according to the present invention consists only of the first structural component, as the second ceramic component has completely dissolved.

The bone substitute of the present invention, in at least one version or in its full bioinduction and bioconduction potential, becomes during implantation the biomaterial in this third and final period of time.

During the second period of time, the second ceramic component is present in increasingly smaller percentages, as it dissolves and therefore comes out of the first structural component and is eluted with the fluids, for example biological.

Furthermore, during the second and third periods of time, as the second ceramic component dissolves and/or solubilises, leaving empty spaces corresponding to the micro-cavities and macro-cavities in which it was contained, it begins inside the first structural component the bone regrowth of the bone tissue surrounding the implant. Furthermore, it has been seen that - thanks to the specific structure of the biomaterial according to the present invention - blood circulation is also recreated within the first structural component, ensuring perfect adhesion of the regenerated bone tissue with the material that constitutes the first structural component. Indeed, during the third period of time, it is almost no longer possible to distinguish the material of the first structural component from the newly formed bone tissue. Ultimately, once completely abandoned from the calcium salt, the biomaterial remains chemically identical to the first component, for example to bone cement. However, from histological observations it can be seen that the biomaterial according to the present invention maintains an intimacy with the bone tissue that is far superior to traditional bone cement. This is explained because it is a completely "functioning" tissue, as, as mentioned, it is perfused of fluids, blood and substances useful for its development and growth. Therefore, the second and third time periods occur within the human body following the implantation which, in its initial configuration, is found as defined for the first time period, i.e. consisting of the first structural component and the entire second ceramic component.

The second ceramic component, as mentioned, is reabsorbable. **In** fact, it is reabsorbed by the human body once dissolved and/or solubilized. However, even once completely solubilized, it leaves clear traces of its past presence. These traces can be found on the surface and in the depths of the structural component in the form of fine roughness that covers the canaliculi, the micro-cavities and the macro-cavities. This surface modification acts as a real "texture" which greatly increases the affinity between the biological tissue and the synthetic one, which is precisely the structural component.

The biomaterial and/or bone substitute according to the present invention, in fact, comprises a surface suitable for coming into contact with the bone tissue in use in the implantation site, wherein this surface includes an external surface of the biomaterial and/or bone substitute and an internal surface, given by the walls that constitute and/or surround the porosity.

This surface is wrinkled and/or textured.

In fact, it is the surface of the structural component (for example PMMA) which, once freed from all the second component or calcium salt, presents all the surfaces, including those of both the micro and macro cavities and the canaliculi, rough and/or roughened and/or textured. This roughness is formed by the powder of the second component, which remains as a sort of imprint after its dissolution.

This roughness, as mentioned, has a positive function towards the biological (bone) tissue which is very similar to the synthetic material (biomaterial or bone substitute according to the present invention), remaining intimately contiguous with it.

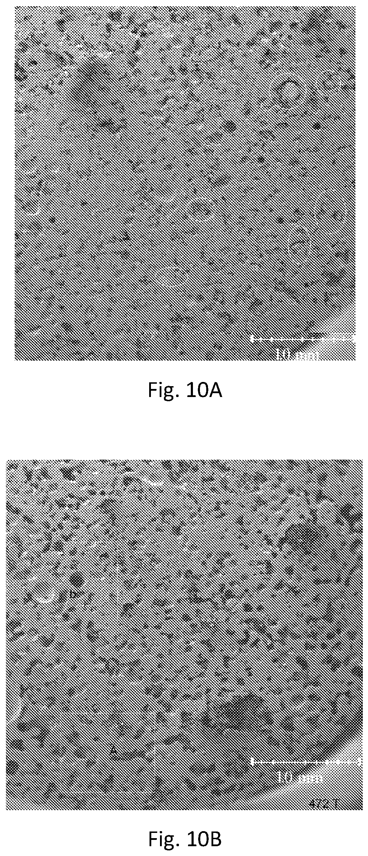

In figure 10A you can see the structure of the biomaterial before implantation: the rich network of canaliculi and the porosity are macroscopically evident both in the form of micro-cavities, determined by the powder with a diameter of less than 100 microns, and in the form of macro- cavities, determined by granules larger than 200 microns, powder and granules actually present in the micro- and macro-cavities, in addition to the surface roughness of the material.

In Figure 10B, the same material is shown as in Figure 10A but after the powder and granules of second material or TCP have been dissolved. The removal of the second material or TCP enhances and determines both the micro- and macro-porosity. In this phase, the roughness is much deeper and has a strong bioaffine action on the entire bone-biomaterial interface.

This does not happen, for example, with a bone cement having the same nature as the first component but without the second solubilized component, as the bone cement is very compact, and its surface is smooth.

The wrinkled and/or textured surface includes a series of grooves and/or indentations, of a more or less regular curvilinear or spherical cap shape. These grooves and/or indentations have a dimension of less than 100 microns, as they are left by the imprint of the powder particles, for example, of the second component, which in turn have a dimension of less than 100 microns.

In at least one version of the invention, this surface does not have grooves and/or indentations that are very regular and distinguishable from each other, but rather at least partially overlapped, and form exactly a wrinkled surface, created by the presence of valleys or protrusions and grooves and/or indentations.

In another version, however, the grooves and/or indentations are clearly distinguishable as they are further apart from each other.

Naturally, the duration of the second time period can be very different depending on the type and speed of solubilization of the material that constitutes the second ceramic component.

For example, according to a version of the present invention (called Type 0 if desired), the biomaterial containing calcium sulphate salts presents an early solubilisation, for example 3-6 months; according to a further version (called Type 1 if desired), the biomaterial containing calcium carbonate salts has rapid solubility, for example 8-12 months; according to a version of the present invention (called Type 2 if desired), the biomaterial containing tricalcium phosphate salts has a slow solubility, for example 12-24 months; finally, in a further version (if desired called Type 3), the biomaterial containing hydroxyapatite salts has a very slow solubility, for example more than 24 months.

The presence of micro-cavities and canaliculi, both having dimensions smaller than 100 microns and being variously interconnected, gives the biomaterial according to the present invention capillary properties. Therefore, in use, water or biological fluids are drawn into the biomaterial, together with any substances capable of promoting or facilitating bone regrowth, and this determines bone regrowth right up into the macro-cavities of the entire biomaterial.

Furthermore, as will be better explained later, the biomaterial presents a homogeneous and regular capillarity, distributed without discontinuity throughout the entire volume of the biomaterial. This result is better than currently known materials.

During the manufacturing phase of the biomaterial according to the present invention, the first structural component is in the form of a powder part and a liquid part.

The powder part may comprise at least one of the above materials in powder form.

The powder part can have a grain size between 0.1 micron and 500 microns.

The powder part can have a various morphology, and/or be made up, for example, of biocompatible polymethylmethacrylate and/or copolymers of methylmethacrylate, etc.

Furthermore, the powder portion may comprise a polymerization catalyst for example Benzoyl Peroxide.

For example, in the case of acrylic resins, the liquid part includes a monomer, for example MMA which will polymerize the powder polymer. The liquid part can also comprise a stabilizer, for example hydroquinone, and a polymerization accelerator, such as for example NNDT (NN dimethyl-p-toluidine), for example in an amount ranging from 0.000% to 5%.

For example, considering the biomaterial in solid form according to the present invention, the polymerization accelerator, such as NNDT, may not be present. In this case, in fact, it is possible to polymerize and/or solidify the biomaterial (and/or the first structural component, for example MMA or PMMA) by heat, for example in a stove, effectively eliminating the need for this substance.

However, this does not happen when considering the biomaterial in fluid or paste form of prior art (for example those forms made directly by the surgeon), as in this case the polymerization accelerator must be present.

The powder part may comprise at least one of the above materials in powder form.

For example, in the case of acrylic resins, the powder part can include a polymer powder part, capable of reacting with the liquid part and forming the indicated material by polymerization and/or solidification. For example, the powder part can comprise polymethyl methacrylate (PMMA) and/or copolymers of methyl methacrylate (biocompatible) and BP (Benzoyl Peroxide, an indispensable catalyst in at least one version of the present invention).

The BP can be already incorporated into the PMMA or added as a free powder component, mixed with the PMMA.

To have a robust PMMA the following requirements must be met:
1. eliminate structural imperfections such as the presence of air bubbles. In fact, the larger the air bubbles, the worse the mechanical performance.
2. Bring the conversion (polymerization) percentage to the maximum possible. In other words, it would be useful to try to transform all MMA into PMMA.

To do this, or rather for point 1, it is not sufficient to remove the air by applying vacuum. In fact, since the bubbles are dispersed in a fluid with progressive and incipient viscosity, the vacuum does nothing but increase the diameter of the bubbles and at the same time evaporates the liquid component MMA which has a low vapor pressure, thus effectively increasing the number of bubbles overall.

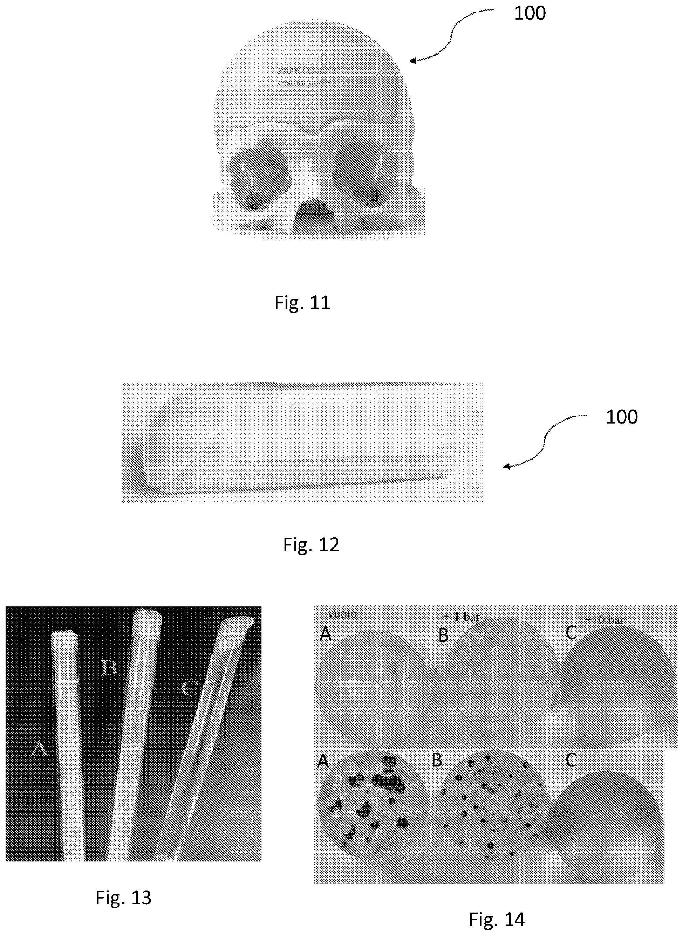

It has been seen, however, that by increasing the ambient pressure, the diameter of the bubbles is reduced to invisible dimensions. Figure 13 compares three specimens made with the same resin (first component, for example PMMA) polymerized under vacuum (A), at ambient pressure (B) and at a pressure of 10 bar (C). Observing, specimen A contains the maximum number of bubbles, specimen B contains but fewer than specimen A and in specimen C the resin is apparently vitrified because there are no obvious bubbles.

In figure 14, the sections of the specimens indicated above can be seen and the presence of large bubbles in specimen A, small bubbles in specimen B and no bubbles in specimen C, comprising the first component according to the present invention, can be seen.

To increase the conversion rate of MMA to PMMA (point 2 mentioned above) after polymerization at room temperature, the specimen is moderately heated. In this way the MMA molecules that are still free but imprisoned and immobilized in the PMMA polymer begin to move again due to the effect of the thermal energy and polymerize in turn. A polymer with a high conversion percentage has mechanical performance of the order of 10% more than the same polymer with a lower conversion percentage.

Obviously where there are bubbles there is no material and this affects the weight of the specimen. See for example the data reported in Table 4, in which six specimens for each type of specimen are compared.

**Table 4: Weight in grams of specimens A, B and C**

| | **Specimen A** | **Specimen B** | **Specimen C** |
|---|---|---|---|
| 1 | 0.6847 | 0.8134 | 0.8561 |
| 2 | 0.6026 | 0.7939 | 0.8472 |
| 3 | 0.5518 | 0.7853 | 0.8318 |
| 4 | 0.5620 | 0.8055 | 0.8212 |
| 5 | 0.6321 | 0.7973 | 0.8341 |
| 6 | 0.6436 | 0.7978 | 0.8224 |
| **Average** | **0.5961** | **0.7988** | **0.8355** |

From the data indicated above it emerges that the presence of the bubbles determines a weight of approximately 70% for specimen A compared to specimen C and of approximately 95% for specimen B compared to specimen C.

Table 5 below also shows the values of the compressive resistance exerted by the specimens A, B and C indicated above (six specimens for each type of specimen are compared).

**Table 5: Compressive strength in MPa of specimens A, B and C**

| | **Specimen A** | **Specimen B** | **Specimen C** |
|---|---|---|---|
| 1 | 64.45 | 120.31 | 144.67 |
| 2 | 73.55 | 127.92 | 145.07 |
| 3 | 49.38 | 128.66 | 141.29 |
| 4 | 58.90 | 128.09 | 145.44 |
| 5 | 77.35 | 130.96 | 137.81 |
| 6 | 77.11 | 128.91 | 131.65 |
| **Average** | **66.79** | **127.48** | **140.99** |

From the data indicated above it emerges that the presence of the bubbles determines a force of approximately 47% for specimen A compared to specimen C and of approximately 90% for specimen B compared to specimen C.

Naturally, referring these specimens to the present invention, it must be considered that the material constituting the specimens corresponds to the first component of the material according to the present invention, in detail only specimen C is made according to the innovative method envisaged and described here. In the samples A, B and C, however, the second component is missing, which is instead present in the biomaterial according to the present invention, and consequently the micro- and macro-cavities determined during the polymerization by its presence are missing.

The compressive strength trend, however, is also valid considering the presence of the second component, even if the values change as a consequence of its presence. In any case, the biomaterial polymerized at positive pressure higher than ambient pressure has improved mechanical characteristics of compressive strength compared to the polymerization of the same material at ambient pressure or in vacuum.

The method for producing the biomaterial according to the present invention mainly presents the following steps:
provide the material that constitutes the first structural component in the form of a powder part,
supply the material that constitutes the second ceramic component in the form of powder and granules,
mix the second ceramic component together with the powder part of the first ceramic component, so as to obtain a mixture of particles with homogeneous distribution of the two components,
provide the material that constitutes the first structural component in the form of a liquid part,
add the liquid part of the first structural component to the particle mixture,
mix the liquid part and the mixture of particles so that the liquid part reacts with the powder part of the first structural component to form a biomaterial in fluid form within which the second ceramic component (which is in solid form) is homogeneously distributed,
polymerize and/or solidify, for example in a special mold, this biomaterial in fluid form, until it solidifies.

The method according to the present invention advantageously includes a step of subjecting the biomaterial in fluid form, during the polymerization and/or solidification step, to a positive pressure of between +4 and +12 bar.

In this way, an increase in mechanical performance is obtained, while maintaining the capillarity properties unchanged.

As can be seen from the present description, there is no interaction of any bond between the second component (for example TCP) and the first structural component (for example PMMA). This also explains the mechanical compression behaviour of the biomaterial. In fact, the biomaterial including the second component (e.g. TCP) offers - in at least one version - superior performance of approximately only 10% compared to the same biomaterial in which the second component has been removed. The data confirm that, during the compression test, the biomaterial is compressed and/or crushed: if the second component is present, the latter offers a modest resistance, while if the second component has been removed or is absent, the resistance of the biomaterial is only slightly inferior. As a consequence, the biomaterial mechanical performances are mainly determined by the first component and by its realization manner, in at least one version of the present invention.

Micro-porosities and macro-porosities are present in the newly formed biomaterial, therefore they can coexist with the second component.

The dimensions of the micro-cavities and/or macro-porosities are stable and immutable. For example, they do not change when the biomaterial is implanted in vivo. What is formed with the dissolution and/or removal of the second component is therefore the patency of the porosity or at least of the micro-cavities and/or macro-cavities of the biomaterial according to the present invention.

According to an embodiment, the step of polymerizing and/or solidifying, as well as that of subjecting the biomaterial undergoing polymerization and/or solidification to a positive pressure, can take place in an autoclave.

The increase in pressure compared to atmospheric pressure can be caused by air pressure.

In this way, the biomaterial in fluid form and in the polymerization and/or solidification phase, when subjected to positive pressure, is compacted and the air bubbles that remain trapped in the material during polymerization and/or solidification are eliminated or considerably reduced.

Naturally, the presence of air bubbles in the biomaterial produces a negative effect both in terms of capillarity, as they actually interrupt the canaliculi and/or micro-porosities present in the biomaterial, and in terms of mechanical performance, because they actually interrupt the structure of the material through the presence of empty spaces caused by the air bubbles themselves.

By its nature, the air trapped in the powder at the origin and that introduced due to the powder-liquid mixing step is a closed porosity, i.e. the individual bubbles are isolated and constitute areas of weakening of the material. Air bubbles undermine the solidity of the material and to increase the mechanical performance of the material it is necessary to eliminate or at least reduce the air bubbles as much as possible.

Furthermore, even moisture droplets present in the biomaterial will avoid aggregating by remaining smaller thanks to pressurization. Smaller droplets correspond to smaller porosities with less reduction in the fatigue performance of the biomaterial. It follows that the biomaterial according to the present invention will last longer in vivo, once implanted in the human body.

In an alternative version, to the positive pressure a step of subjecting the biomaterial in a liquid or fluid state, undergoing polymerization and/or solidification, at a high temperature is added, for example a temperature higher than 50°C up to approximately 90°C or in any case lower than 100°C. In this case, it is possible, for the liquid part of the first structural component, not to contain polymerization accelerator or NNDT. In fact, as indicated previously, heat increases molecular agitation and consequently increases the conversion percentage of the monomer into the polymer and therefore - in fact - increase the mechanical performance.

In yet another version of the present invention, the step of subjecting the biomaterial to a positive pressure is contemporary with the step of subjecting the biomaterial to a high temperature.

In an alternative version, the step of subjecting the biomaterial to a high temperature can occur after the step of subjecting the biomaterial to a positive pressure.

In yet another version, the biomaterial is first subjected to a positive pressure and, after a certain period of time, even a few hours if desired, it is also subjected to an elevated temperature (maintaining positive pressure).

The total time of this step of subjecting the biomaterial to a positive pressure and/or a high temperature can last up to 12 hours for example.

The polymerized and/or solidified biomaterial is therefore compact.

One step of the above method comprises providing a certain level of moisture, possibly in the form of water or an aqueous solution or saline solution, and adding such level of moisture to the particle mixture and/or the first structural component and/or the second ceramic component.

In a version of the invention, preferred if desired, the level of humidity is added to the second ceramic component, obtaining a second wet ceramic component. This second wet ceramic component is then added to the powder part of the first structural component, before the addition of the liquid part of the first structural component.

The presence of humidity determines, during the polymerization and/or solidification step, the formation of the canaliculi of the first structural component and/or facilitates the mixing steps.

The biomaterial according to the present invention can therefore comprise a variable percentage humidity level, possibly in the form of water or aqueous solution or saline solution.

According to at least one version of the present invention, the biomaterial according to the present invention comprises (at least during its initial manufacturing phase) an overall liquid portion comprising or consisting of the specific humidity level and the liquid part of the first structural component.

In particular, this overall liquid portion comprises 25-45% of the humidity level, for example saline solution, and 75-55% of the liquid part of the first structural component.

According to at least one version of the present invention, the biomaterial according to the present invention comprises an overall solid portion comprising or constituted (at least during its initial manufacturing phase or during the first period of time after implantation) by the powder part of the first structural component and from powder and granules of the second ceramic component.

In particular, this overall solid portion includes 20-80% of the powder part of the first structural component and 80-20% of the second ceramic component.

According to an example of the present invention, the overall liquid portion corresponds to approximately 30-70 grams and is added to 100 grams of overall solid portion.

The biomaterial according to at least one version of the present invention is a composite of solid substances made up of a first structural component and a second structural component (e.g. PMMA and TCP) which must coexist with mutually immiscible liquid substances such as water and for example the solvent or MMA (methyl methacrylate).

The water or humidity can be dispersed during mixing partly in the form of droplets which will give rise to canaliculi and partly adsorbed on the second component or TCP. During mixing, air may also be incorporated which is dispersed, for example, in the form of bubbles.

A bullous material is a discontinuous material and inherent mechanical weakness. Therefore, thanks to the pressurization of the present method, the structural integrity of the biomaterial according to the present invention is restored and improved.

In fact, any bubbles of air and/or water or humidity present in the fluid mass are compressed, considerably decreasing their volume and/or size. This gives greater mechanical performance to the biomaterial.

### Experimental tests

In fig. 3 shows, by way of example, a section of the femoral condyle of an adult pig in which the specimen A1 in biomaterial according to the present invention had been inserted 12 months earlier (for example of Type 2, including TCP and for example in which the first component structural is an acrylic resin and/or PMMA). Specimen A1 before implantation has a white color.

The reference number 2 in fig. 3 indicates specimen A1 but, since it is implanted, it no longer has a white color but rather takes on the same color as the surrounding spongy bone (indicated with the reference number 1 in fig. 3). The biomaterial is rich in marrow fat and blood capillaries, because it has been invaded by them. The spongy bone 1 in fig. 3 intimately surrounds specimen A1, indeed it penetrates into it and has a bright vermilion colour, demonstrating the fact that it is a living tissue that regenerates without being disturbed in the slightest by the presence of the biomaterial. Even the cortical bone (indicated with the reference number 3 in fig. 3) adapts its remodelling to the presence of the biomaterial of specimen A1, without the interposition of fibrotic membranes.

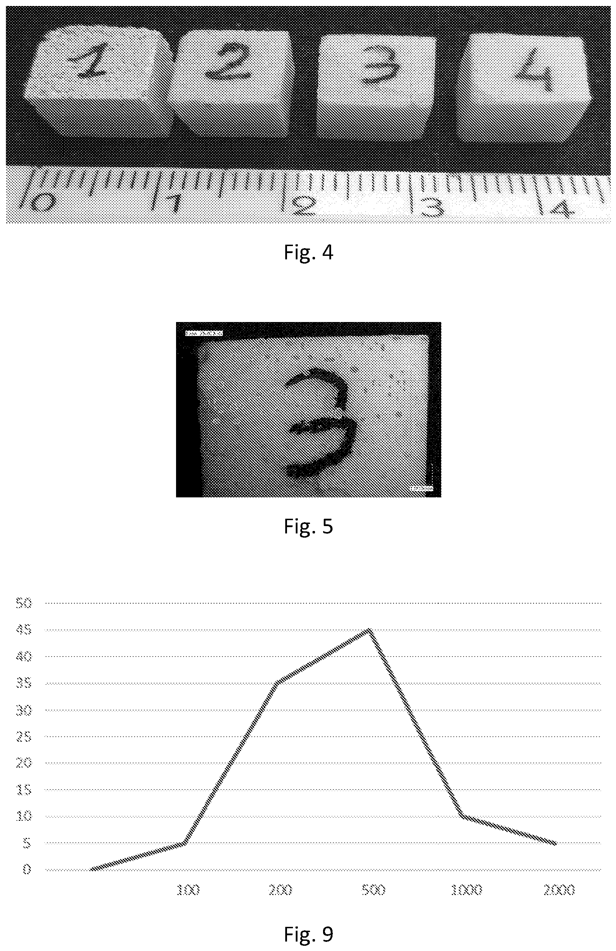

Figure 4 illustrates four specimens (having cubic conformation) subjected to performance comparison in which:
the specimen indicated with no. 1 comprises a porous ceramic material made up of hydroxyapatite and TCP;
the specimen indicated with no. 2 includes the biomaterial according to the present invention (for example an acrylic resin and/or PMMA in combination with TCP), in which both the first structural component and the second ceramic component are still present;
the specimens indicated with no. 3 and no. 4 include the biomaterial according to the present invention, in which the second ceramic component has been reabsorbed and is therefore no longer present (in this mode the biomaterial is also called demineralised).

Specimens no. 3 and 4 in fig. 4 simulate the long-term in vivo conditions of the biomaterial according to the present invention, i.e. when in use, for example biological liquids, have completely dissolved the second ceramic component, in the specific example the TCP.

Figure 5 shows an image of specimen no. 3 in fig. 4 with some pores digitally highlighted. This confirms the porosity of the demineralized biomaterial. As can be seen, this porosity is comparable to that of specimen no. 1.

Figure 6 illustrates the mechanical characteristics compared to specimens no. 1 and no. 3 indicated above in the figure. 4.

In particular, we note the two metal plates of the compression testing machine which crush and break specimen No. 1 made entirely of ceramic (on the left) while they crush and simply deform specimen No. 3 (on the right) made in an example of the biomaterial according to the present invention. It is noted that the crushing leads to almost explosive brittle failure with multiple fragments detaching from specimen no. 1. Specimen no. 3, however, noticeably deforms under the strong load, which is at least double that supported by the ceramic of specimen no. 1, but remains compact, no fragments are released.

From a surgical point of view, this clear difference means that, when a ceramic insert such as the one corresponding, for example, to specimen no. 1, is implanted, it is exposed to the risk for a long time (until it is completely dissolved). of fragile rupture with the consequent emission of many fragments which can migrate into the biological fluids and collect in the serous bags and create bursitis, foreign body arthritis with degeneration of the joint, etc. On the contrary, the biomaterial according to the present invention, which is the one corresponding, for example, to specimen no. 3, can deform under the mechanical load - even high - but, by not losing fragments, it will not produce damage to the tissues or joints close to the implant site.

The results of the compression test can be viewed in the graph in figure 7. Furthermore, by converting the raw data of the load and the consequent stroke of this graph, the following results can be highlighted:

| **Specimen** | **Maximum compressive strength (MPa)** | **Section (mm²)** |
|---|---|---|
| No. 1 | 22.98 | 93.51 |
| No. 2 | 71.53 | 81.02 |
| No. 3 | 62.92 | 90.80 |
| No. 4 | 55.27 | 85.26 |

Specimen no. 1 in fig. 4 has a very low compressive strength of approximately 23 MPa, specimen no. 2 in fig. 4 has the maximum compressive strength, of approximately 72 Mpa, because the biomaterial is still composed - in addition to the first structural component - also of the entire second ceramic component which fills the cavities or porosities giving a high resistance to crushing. Specimens no. 3 and 4 in fig. 4 have a compressive strength of approximately 63 MPa and 55 MPa respectively. Specimens no. 3 and 4 in fig. 4, being demineralised, no longer have the second ceramic component either in the form of powder or in the form of granules in the cavities of the first structural component, and therefore fail earlier than specimen no. 2. These specimens however have a compressive strength which is at least about 2.5 as high when compared t the specimen no. 1.

The biomaterial according to the present invention includes a compressive strength higher than 55 MPa, or higher than 60 MPa, or higher than 70 MPa, depending on the degree of dissolution of the second ceramic component with respect to the first structural component which, as mentioned, it is permanent and insoluble.

The compressive strength data were measured in accordance with the ISO5833 standard, second edition dated 05-01-2002, specifically in the part called "Determination of compressive strength of polymerized cement".

This test involves the creation of cylinders of material to be tested (dimensions approximately 12 mm in length by 6 mm in diameter which are applied on a test machine capable of applying and measuring a compressive force (load) of at least 4 kN, equipped with systems capable of recording the load in relation to the movement of the crosspiece or crosshead of the cylinder.

The cylinder is placed in the test machine and the machine is operated to produce a displacement curve with respect to the load, using a constant crosspiece speed. The test ends when the cylinder fractures or when the upper yield point is exceeded.

For each cylinder, for the purpose of calculating the compressive strength, the force applied to cause the fracture or the load at 2% of the offset ("2% offset load") or the load at the upper sliding limit (whichever happens first), divide the force by the area of the original crosspiece (in mm²) of the cylinder and the quotient is expressed as compressive strength, in Mpa.

The test is carried out by maintaining the material to be tested and the equipment at a temperature of 23±1°C; the cylinders are tested approximately 24 hours after mixing the material to be tested.

Experimental data have demonstrated that the capillarity of a sample of biomaterial polymerized and/or solidified at positive pressure, for example at +7 bar (for example at room temperature or equal to 23°C), is improved compared to the same material polymerized and/or or solidified at atmospheric pressure (1 bar and at room temperature or 23°C).

Furthermore, the speed of absorption of liquids by capillarity of a sample of biomaterial polymerized and/or solidified at positive pressure, for example at +7 bar, is also improved compared to the same material polymerized and/or solidified at atmospheric pressure.

Furthermore, thanks to the notable reduction in the spaces occupied by air bubbles and/or moisture droplets and/or the notable reduction in their size, the absorption of liquids by capillarity is much more regular and homogeneous/uniform in a sample of biomaterial polymerized and/or solidified at positive pressure, for example at +7 bar, compared to the same material polymerized and/or solidified at atmospheric pressure.

Further compression tests were then carried out on the polymerized and/or solidified biomaterial according to the present invention, for example on specimens having dimensions 6.8 mm x 12 mm.

In particular, while known types of materials have a compressive strength of around 35-36 MPa, the polymerized and/or solidified biomaterial according to the present invention has an average compressive strength equal to 60 MPa, or greater than 60 MPa, or greater than 61 MPa, with a standard deviation of approximately 3 MPa. The compressive strength can reach values even higher than 67-75 MPa.

According to at least one version of the present invention, these data were carried out on samples in which the first structural component is formed by an acrylic resin and/or polymethylmethacrylate (PMMA) containing powder and granules of the second ceramic component, in particular TCP.

These data are proof that the biomaterial according to the present invention has improved mechanical and capillarity characteristics compared to prior art materials or to the same material polymerized and/or solidified at atmospheric pressure.

The biomaterial according to the invention is used as a reconstructive material, acting as a bone substitute or filling material, and placed in a cavity or gap or bone atrophy. As seen, thanks to the properties of the material indicated above, this gap or atrophy can also be of considerable size. Furthermore, compared to known materials, the biomaterial according to the present invention is able to improve and facilitate the reconstruction of the bone tissue surrounding the implant site, also with the formation of a perfectly integrated and functioning blood circulation, even within the biomaterial itself.

The biomaterial according to the present invention is capable of absorbing various types of fluids, gaseous substances and/or water or other biological liquids and/or drugs and medicinal substances by capillarity. This biomaterial is then able to release these substances outside again and make them available inside.

By virtue of its porous nature and its ability to absorb liquids by capillarity, the biomaterial according to the present invention can therefore be advantageously used as a drug release system (Drug Delivery System), in order to contain and deliver active ingredients also with drug function in the site of placement.

In a further embodiment, the biomaterial described above can be added with radiopaque materials, for example barium sulphate and/or other known radiopaque materials.

The biomaterial or bone substitute according to the present invention comprises or can be added with at least one pharmaceutical or medical substance. Such at least one pharmaceutical or medical substance can initially be present in the first structural component and/or in the second ceramic component and/or can subsequently be introduced into the biomaterial through impregnation, addition, spraying, etc. In this way, the biomaterial according to the present invention can help, for example, to fight the infection present in the bone and/or tissues adjacent to the implantation site.

Such at least one pharmaceutical or medical substance can include, in one version of the invention, at least one antibiotic, such as for example gentamicin, vancomycin, tetracycline, etc. or combinations thereof.

Such at least one pharmaceutical or medical substance can be originally present in the biomaterial according to the present invention, for example it can be incorporated into it by mixing it in the manufacturing phase with the first structural component and/or with the second ceramic component.

Alternatively, the biomaterial or bone substitute may be originally free of at least one pharmaceutical or medical substance and be immersed in a solution comprising such at least one pharmaceutical or medical substance by the surgeon prior to implantation. In this case, the biomaterial absorbs such at least one substance before implantation and releases it into use after implantation.

According to an even further version, the biomaterial or bone substitute can comprise at least one (first) pharmaceutical or medical substance and, before implantation, the surgeon can decide to add at least one (second) pharmaceutical or medical substance by impregnation and/or immersion, the same or different from the first, depending on the specific surgical needs and of the patient.

The present invention also refers to a system in which the biomaterial according to the present invention is present both in solid and fluid form. In detail, the biomaterial in solid form, as previously described, can be in use positioned at the implant site and the biomaterial in fluid form can be used, since it is hardenable, to fix the biomaterial in solid form in position. In this way, the porosity (given by canaliculi, micro-cavities and macro-cavities) of the biomaterial in solid form finds spatial and fluid continuity with the porosity (given by canaliculi, micro-cavities and macro-cavities) of the biomaterial in form fluid, after hardening and/or polymerization and/or solidification of the same.

Differently from what would happen with the use, for example, of traditional bone cement, which does not have adequate porosity and therefore can form a fibrous sheath at the interface with the bone tissue, fixing the biomaterial in solid form with the biomaterial in fluid form and by allowing the latter to solidify, a continuity of porosity is determined throughout the implanted material, further facilitating bone regrowth.

The biomaterial in fluid form can be the same (except for being in the fluid state) as the biomaterial in solid form or it can vary from the latter in terms of quantity and/or type of the second ceramic component.

Alternatively or in addition, the biomaterial in fluid form can vary compared to the biomaterial in solid form in terms of quantity and/or type of material that comprises the first structural component.

Alternatively or in addition, the biomaterial in fluid form can vary compared to the biomaterial in solid form due to the quantity and/or type of pharmaceutical or medical substances and/or radiopaque materials.

The biomaterial in fluid form (solidifiable) therefore acts as a glue for the biomaterial in solid form.

The biomaterial in fluid form is in the form of an injectable paste, for example using an extrusion syringe, or applicable for example through the use of a spatula, via the standard methods currently used for known materials.

The fact that the biomaterial can be present in this fluid form is conferred by the ability of the first structural component to be in fluid form, and to solidify after its application in a pre-established, relatively short time of a few minutes, compared to known materials.

In this case, the biomaterial in fluid form can contain a polymerization accelerator in its composition while the biomaterial in solid or preformed form can present it or not, for its realization.

## Claims

1. Composite biomaterial, biocompatible, osteoinductive and/or osteoconductive, suitable during use to be used as a bone substitute for filling lesions, gaps or bone cavities or for the functional restoration of tissues in which the replacement or restoration of bone tissue is necessary, comprising a first structural component, of a permanent and non-absorbable type in use in the human body, wherein said first structural component comprises an organic, plastic and/or polymeric material, and a second ceramic component, of a soluble or solubilizable type in use in contact with a liquid, for example a biological liquid, wherein said first structural component comprises an interconnected porosity comprising pores and/or canaliculi with a diameter of less than 100 microns, wherein said second ceramic component, housed in said porosity, includes at least one calcium salt, wherein said second ceramic component is in the form of a powder with a size of less than 100 microns and granules with a size of between 100 microns and 2000 microns, wherein said composite biomaterial is obtained by the method of any one of claims 10 to 14, **characterized by the fact that** said biomaterial is in the form of a preformed solid and in which said biomaterial has a compressive strength greater than 55Mpa, measured in accordance with the ISO5833 standard, second edition dated 01- 05-2002.

2. Biomaterial according to claim 1, wherein said biomaterial is compact and/or has a three-dimensional spherical, hemispherical, polyhedral, parallelepiped, hexahedron, truncated cone or truncated pyramid, cone or pyramid, paralleliform, chock-shaped, wedge-shaped conformation, or an irregular three-dimensional shape.

3. Biomaterial according to claim 1 or 2, having a compressive strength greater than 60 MPa, or greater than 70 Mpa or greater than 67-75 Mpa, measured in accordance with the ISO5833 standard, second edition dated 01-05-2002.

4. Biomaterial according to any of the previous claims, wherein said granules have a size of between 100 microns and 500 microns or between 200 microns and 500 microns or between 200 micron and 2000 micron and/or wherein said calcium salt of said second ceramic component comprises a compound based on calcium phosphate (CP), a calcium sulphate (CS), calcium carbonate (CC), in wherein said calcium phosphate compound comprises at least one or more of: calcium dihydrogen phosphate monohydrate (MCPM), calcium dihydrogen phosphate anhydrous (MCPA), calcium monohydrogen phosphate dihydrate (DCPD), calcium monohydrogen phosphate anhydrous (DCP), octacalcium phosphate (OCP), alpha-tricalcium phosphate (α-TCP), beta-tricalcium phosphate (β-TCP), hydroxyapatite (HA) tetracalcium phosphate (TTCP).

5. Biomaterial according to any of the previous claims, wherein said second ceramic component is homogeneously distributed throughout the volume occupied by said biomaterial, at least for a first period of time in use after the implant.

6. Biomaterial according to the previous claim, capable, after said first period of time in use after the implant, of no longer comprising said second ceramic component, since said second ceramic component in use has dissolved and/or solubilized.

7. Biomaterial according to any of the previous claims, wherein said first structural component comprises a porosity further comprising micro-cavities with a size of less than 100 microns and macro-cavities with a size of between 100 and 500 microns or between 200 microns and 500 microns or between 300 micron and 500 micron or between 100 micron and 2000 micron or between 200 micron and 2000 micron or between 300 micron and 2000 micron or more than 250 micron and up to 2000 micron or more than 500 micron and up to 2000 micron, wherein said porosity is interconnected and homogeneously distributed throughout the volume occupied by said biomaterial and/or in which said second ceramic component is housed, in use at least for said first period of time in use after the implant, inside said porosity formed by said micro-cavities and said macro-cavities of said first structural component.

8. Biomaterial according to any of the preceding claims, wherein said organic, plastic and/or polymeric material of said first structural component comprises or is constituted by a plastic material, a plastic or acrylic or methacrylic resin, or a plastic or acrylic polymer or a cement bone made of polymethyl methacrylate (PMMA) and/or a copolymer based on methyl methacrylate or a thermoplastic polymer, polyethylene, polypropylene, polyester, a thermoformable polymer or other similar materials.

9. Biomaterial according to any of the previous claims, comprising a humidity level, in the form of water or aqueous solution or saline solution and/or at least one radiopaque material, and/or a pharmaceutical or medical substance, such as at least one antibiotic.

10. Method for making a composite biomaterial according to one or more of the previous claims, wherein said composite biomaterial is biocompatible, osteoinductive and/or osteoconductive, suitable in use to be used as a bone substitute for filling lesions, gaps or bony cavities or for the functional restoration of tissues in which the replacement or restoration of bone tissue is necessary in use, comprising a first structural component, of a permanent and non-absorbable type used in the human body, and a second ceramic component of a soluble or solubilisable type in use in contact with a liquid, for example a biological liquid, comprising the following steps:
providing said first structural component comprising an organic, plastic and/or polymeric material, wherein said first component comprises a powder part and a liquid part, wherein said powder part comprises a polymeric powder part, capable of reacting with said part liquid,
provide said second ceramic component comprising at least one calcium salt, wherein said second ceramic component is in the form of a powder with a size of less than 100 microns and granules with a size of between 100 microns and 2000 microns,
mixing said second ceramic component together with said powder part of said first ceramic component, so as to obtain a mixture of particles with homogeneous distribution of the two components,
add said liquid part of said first structural component to said mixture of particles,
mixing said liquid part and said mixture of particles so that said liquid part reacts with said powder part of said first structural component to form a biomaterial in fluid form within which said second ceramic component is homogeneously distributed,
polymerize and/or solidify, for example in a special mold and/or in an oven and/or in an autoclave, said biomaterial in fluid form, until it solidifies,
subjecting said biomaterial in fluid form to a positive pressure between +4 and +12 bar, thus obtaining a compaction of the biomaterial and a reduction in the volume and/or size of any bubbles of air and/or of water or humidity present and possibly trapped in the biomaterial during said step of polymerization and/or solidification,
obtaining said biomaterial in the form of a preformed solid, having a compressive strength greater than 55Mpa, measured in accordance with the ISO5833 standard, second edition dated 01-05-2002.

11. Method according to claim 10, wherein said step of subjecting to a positive pressure occurs during said step of polymerizing and/or solidifying.

12. Method according to claim 10 or 11, wherein said subjecting step further comprises subjecting said biomaterial in fluid form to an elevated temperature, less than 100°C.

13. Method according to one or more of claims 10 to 12, wherein said step of providing said second ceramic component comprises providing said second ceramic component in the form of granules with a size of between 100 microns and 500 microns or between 200 microns and 500 microns or between 300 and 500 microns or between 200 microns and 2000 microns or between 300 microns and 2000 microns or more than 250 microns up to 2000 microns or more than 500 microns up to 2000 microns and/or comprising a step of providing a certain level of humidity, in the form of water or aqueous solution or saline solution, and adding said level of humidity to said second ceramic component, obtaining a second wet ceramic component and adding said second wet ceramic component to said powder part of said first structural component, before said step of adding said liquid part of said first structural component.

14. Method according to one or more of claims 10 to 13, comprising a step of adding at least one radiopaque material and/or a pharmaceutical or medical substance, such as at least one antibiotic, to said first structural component and/or to said powder part of said first structural component and/or said liquid part of said first structural component and/or said second ceramic component and/or a step of immersing said biomaterial in a solution comprising at least one pharmaceutical or medical substance.

15. Kit for filling lesions, gaps or bone cavities or for the functional restoration of tissues in which the replacement or restoration of bone tissue is necessary in use, comprising a biomaterial in the form of a preformed solid according to any of claims 1 to 9 and a biomaterial in fluid form, wherein said biomaterial in fluid form comprises a first structural component in solidifiable fluid form and a second ceramic component, wherein said first structural component in fluid form is the same or different than the first structural component of said biomaterial in the form of a preformed solid and said second ceramic component of said biomaterial in fluid form is the same or different than said second ceramic component of said biomaterial in the form of preformed solid.

16. Kit according to the previous claim, wherein said biomaterial in fluid form acts as a glue and adhesive in use in the implantation site for said biomaterial in the form of a preformed solid.

17. Biomaterial according to any one of claims 1 to 9 or kit according to claim 15 or 16, for use as a bone substitute for filling lesions, gaps or bone cavities or for the functional restoration of tissues in which the replacement or restoration of bone tissue are necessary in use, for example for large bone gaps or cavities, large lesions caused by war wounds, large tumors, major trauma, etc.

18. Bone substitute for filling bone lesions, gaps or cavities or for the functional restoration of tissues where replacement or restoration of bone tissue is necessary in use, e.g. for large bone gaps or cavities, large lesions caused by wounds of war, large tumors, major traumas, etc., comprising a composite biomaterial according to any one of claims 1 to 9 comprising a first structural component, of a permanent and non-absorbable type in use in the human body, and a second ceramic component, of the soluble or solubilizable type, wherein said first structural component comprises an organic, plastic and/or polymeric material, wherein said first structural component includes an interconnected porosity comprising pores and/or canaliculi less than 100 microns in diameter, microcavities less than 100 microns in size, and macrocavities, in which at least some of said micro-cavities and said macro-cavities are suitable in use for housing said second ceramic component, wherein said second ceramic component comprises at least one calcium salt and is in the form of a powder with a size of less than 100 microns and granules with a size of between 100 microns and 2000 microns, in which said porosity is homogeneously distributed throughout the volume occupied by said bone substitute, **characterized by the fact that** said biomaterial is in the form of a preformed solid and has a compressive strength greater than 55 Mpa, measured in accordance with the ISO5833 standard, second edition dated 01-05-2002.

19. Bone substitute according to claim 18, wherein said microcavities and said macrocavities are made hollow in use by the solubilization of said second ceramic component, of the soluble or solubilizable type in use in contact with water, a liquid, for example a biological liquid, in which said second ceramic component comprises at least one calcium salt.

20. Bone substitute according to claim 18 or 19, wherein said macrocavities have a size of between 100 and 500 microns or between 200 microns and 500 microns or between 300 microns and 500 microns or between 100 microns and 2000 microns or between 200 microns and 2000 microns or between 300 microns and 2000 microns or more than 250 microns up to 2000 microns or more than 500 microns up to 2000 microns.

21. Bone substitute according to any one of claims 18 to 20, wherein said first structural component comprises a surface adapted to contact the bone tissue in use at the implant site, wherein said surface comprises an external surface of said bone substitute and an internal surface, given by the walls that constitute and/or surround said porosity, in which said surface is wrinkled and/or textured.

## Patentansprüche

1. Biomaterial-Komposit, biokompatibel, osteoinduktiv und/oder osteokonduktiv, das im Gebrauch dazu geeignet ist, als ein Knochenersatz zum Füllen von Läsionen, Lücken oder Knochenkavitäten oder zur funktionellen Wiederherstellung von Geweben verwendet zu werden, worin die Ersetzung oder die Wiederherstellung von Knochengewebe notwendig ist, umfassend eine erste Strukturkomponente, eines permanenten und nicht-resorbierbaren Typs im Gebrauch im menschlichen Körper, worin die besagte erste Strukturkomponente ein organisches, Kunststoff- und/oder polymeres Material, und eine zweite keramische Komponente, eines löslichen oder solubilisierbaren Typs im Gebrauch in Kontakt mit einer Flüssigkeit, zum Beispiel einer biologischen Flüssigkeit, umfasst, worin die besagte erste Strukturkomponente eine miteinander verbundene Porosität, umfassend Poren und/oder Kanälchen mit einem Durchmesser von weniger als 100 Mikrometer, umfasst, worin die besagte zweite keramische Komponente, die in der besagten Porosität aufgenommen ist, mindestens ein Calciumsalz enthält, worin die besagte zweite keramische Komponente in Form eines Pulvers mit einer Größe von weniger als 100 Mikrometer und Granulaten mit einer Größe zwischen 100 Mikrometer und 2000 Mikrometer vorliegt, worin das besagte Biomaterial-Komposit gemäß dem Verfahren irgendeiner der Ansprüche 10 bis 14 erhalten wird, **dadurch gekennzeichnet, dass** das besagte Biomaterial-Komposit in Form eines vorgeformten Feststoffs vorliegt und worin das besagte Biomaterial-Komposit eine Druckfestigkeit von mehr als 55 MPa aufweist, gemessen gemäß der Norm ISO 5833, zweite Ausgabe vom 01.05.2002.

2. Biomaterial nach Anspruch 1, worin das besagte Biomaterial kompakt ist und/oder eine dreidimensionale kugelförmige, halbkugelförmige, polyedrische, parallelepipedische, hexaedrische, kegelstumpfförmige oder pyramidenstumpfförmige, kegel- oder pyramidenförmige, parallelförmige, keilförmige, kuneiförmige Gestalt oder eine unregelmäßige dreidimensionale Form aufweist.

3. Biomaterial nach Anspruch 1 oder 2, das eine Druckfestigkeit von mehr als 60 MPa oder von mehr als 70 MPa oder von mehr als 67-75 MPa aufweist, gemessen gemäß der Norm ISO 5833, zweite Ausgabe vom 01.05.2002.

4. Biomaterial nach irgendeinem der vorangegangenen Ansprüche, worin die besagten Granulate eine Größe zwischen 100 Mikrometer und 500 Mikrometer oder zwischen 200 Mikrometer und 500 Mikrometer oder zwischen 200 Mikrometer und 2000 Mikrometer aufweisen und/oder worin das besagte Calciumsalz der besagten zweiten keramischen Komponente eine Verbindung auf der Basis von Calciumphosphat (CP), ein Calciumsulfat (CS), Calciumcarbonat (CC) umfasst, worin die besagte Calciumphosphatverbindung mindestens eines oder mehreres umfasst aus: Calciumdihydrogenphosphat-Monohydrat (MCPM), wasserfreiem Calciumdihydrogenphosphat (MCPA), Calciummonohydrogenphosphat-Dihydrat (DCPD), wasserfreiem Calciummonohydrogenphosphat (DCPA), Octacalciumphosphat (OCP), Alpha-Tricalciumphosphat (α-TCP), Beta-Tricalciumphosphat (β-TCP), Hydroxyapatit (HA) Tetracalciumphosphat (TTCP).

5. Biomaterial nach irgendeinem der vorangegangenen Ansprüche, worin die besagte zweite keramische Komponente homogen über das gesamte Volumen, das von dem besagten Biomaterial eingenommen wird, mindestens während eines ersten Zeitraums im Gebrauch nach der Implantation, verteilt ist.

6. Biomaterial nach dem vorangegangenen Anspruch, das, nach dem besagten ersten Zeitraum im Gebrauch nach der Implantation, fähig ist, die besagte zweite keramische Komponente nicht mehr zu umfassen, da sich die besagte zweite keramische Komponente im Gebrauch aufgelöst und/oder solubilisiert hat.

7. Biomaterial nach irgendeinem der vorangegangenen Ansprüche, worin die besagte erste Strukturkomponente eine Porosität umfasst, ferner umfassend Mikro-Kavitäten mit einer Größe von weniger als 100 Mikrometer und Makro-Kavitäten mit einer Größe zwischen 100 und 500 Mikrometer oder zwischen 200 Mikrometer und 500 Mikrometer oder zwischen 300 Mikrometer und 500 Mikrometer oder zwischen 100 Mikrometer und 2000 Mikrometer oder zwischen 200 Mikrometer und 2000 Mikrometer oder zwischen 300 Mikrometer und 2000 Mikrometer oder mehr als 250 Mikrometer und bis zu 2000 Mikrometer oder mehr als 500 Mikrometer und bis zu 2000 Mikrometer, worin die besagte Porosität miteinander verbunden und homogen über das gesamte Volumen, das von dem besagten Biomaterial eingenommen wird, verteilt ist und/oder worin die besagte zweite keramische Komponente, im Gebrauch mindestens für den besagten ersten Zeitraum im Gebrauch nach der Implantation, innerhalb der besagten Porosität, die durch die besagten Mikro-Kavitäten und die besagten Makro-Kavitäten der besagten ersten Strukturkomponente gebildet wird, aufgenommen ist.

8. Biomaterial nach irgendeinem der vorangegangenen Ansprüche, worin das besagte organische, Kunststoff- und/oder polymere Material der besagten ersten Strukturkomponente ein Kunststoffmaterial, ein Kunststoff- oder Acryl- oder Methacrylharz oder ein Kunststoff- oder Acrylpolymer oder einen Knochenzement aus Polymethylmethacrylat (PMMA) und/oder ein Copolymer auf der Basis von Methylmethacrylat oder ein thermoplastisches Polymer, Polyethylen, Polypropylen, Polyester, ein thermoformbares Polymer oder andere ähnliche Materialien umfasst oder daraus besteht.

9. Biomaterial nach irgendeinem der vorangegangenen Ansprüche, umfassend einen Feuchtigkeitsgehalt, in Form von Wasser oder einer wässrigen Lösung oder einer Salzlösung und/oder mindestens ein röntgendichtes Material und/oder eine pharmazeutische oder medizinische Substanz, wie beispielsweise mindestens ein Antibiotikum.

10. Verfahren zur Herstellung eines Biomaterial-Komposits nach einem oder mehreren der vorangegangenen Ansprüche, worin das besagte Biomaterial-Komposit biokompatibel, osteoinduktiv und/oder osteokonduktiv ist, das im Gebrauch dazu geeignet ist, als ein Knochenersatz zum Füllen von Läsionen, Lücken oder Knochenkavitäten oder zur funktionellen Wiederherstellung von Geweben, worin die Ersetzung oder die Wiederherstellung von Knochengewebe im Gebrauch notwendig ist, verwendet zu werden, umfassend eine erste Strukturkomponente, eines im menschlichen Körper verwendeten permanenten und nicht-resorbierbaren Typs, und eine zweite keramische Komponente, eines löslichen oder solubilisierbaren Typs im Gebrauch in Kontakt mit einer Flüssigkeit, zum Beispiel einer biologischen Flüssigkeit, umfassend die folgenden Schritte:
Bereitstellen der besagten ersten Strukturkomponente, umfassend ein organisches, Kunststoff- und/oder polymeres Material, worin die besagte erste Komponente einen Pulveranteil und einen Flüssigkeitsanteil umfasst, worin der besagte Pulveranteil einen polymeren Pulveranteil umfasst, der fähig ist, mit dem besagten Flüssigkeitsanteil zu reagieren,
Bereitstellen der besagten zweiten keramischen Komponente, umfassend mindestens ein Calciumsalz, worin die besagte zweite keramische Komponente in Form eines Pulvers mit einer Größe von weniger als 100 Mikrometer und Granulaten mit einer Größe zwischen 100 Mikrometer und 2000 Mikrometer vorliegt,
Mischen der besagten zweiten keramischen Komponente mit dem besagten Pulveranteil der besagten ersten keramischen Komponente, um eine Mischung von Partikeln mit einer homogenen Verteilung der beiden Komponenten zu erhalten,
Hinzufügen des besagten Flüssigkeitsanteils der besagten ersten Strukturkomponente zu der besagten Mischung von Partikeln,
Mischen des besagten Flüssigkeitsanteils und der besagten Mischung von Partikeln, sodass der besagte Flüssigkeitsanteil mit dem besagten Pulveranteil der besagten ersten Strukturkomponente reagiert, um ein Biomaterial in flüssiger Form zu bilden, in dem die besagte zweite keramische Komponente homogen verteilt ist,
Polymerisieren und/oder Verfestigen, zum Beispiel in einer speziellen Form und/oder in einem Ofen und/oder in einem Autoklav, des besagten Biomaterials in flüssiger Form, bis es sich verfestigt,
Aussetzen des besagten Biomaterials in flüssiger Form gegenüber einem positiven Druck zwischen +4 und +12 bar, wodurch eine Verdichtung des Biomaterials und eine Verringerung des Volumens und/oder der Größe irgendwelcher Luftblasen und/oder Wasser oder Feuchtigkeit, die im Biomaterial vorhanden und möglicherweise während des besagten Polymerisations- und/oder Verfestigungsschritts darin eingeschlossen sind, erhalten werden,
Erhalten des besagten Biomaterials in Form eines vorgeformten Feststoffs, mit einer Druckfestigkeit von mehr als 55 MPa, gemessen gemäß der Norm ISO 5833, zweite Ausgabe vom 01.05.2002.

11. Verfahren nach Anspruch 10, worin der besagte Schritt des Aussetzens gegenüber einem positiven Druck während des besagten Schritts des Polymerisierens und/oder Verfestigens stattfindet.

12. Verfahren nach Anspruch 10 oder 11, worin der besagte Schritt des Aussetzens ferner das Aussetzen des besagten Biomaterials in flüssiger Form gegenüber einer erhöhten Temperatur, weniger als 100 °C, umfasst.

13. Verfahren nach einem oder mehreren der Ansprüche 10 bis 12, worin der besagte Schritt des Bereitstellens der besagten zweiten keramischen Komponente das Bereitstellen der besagten zweiten keramischen Komponente in Form von Granulaten mit einer Größe zwischen 100 Mikrometer und 500 Mikrometer oder zwischen 200 Mikrometer und 500 Mikrometer oder zwischen 300 und 500 Mikrometer oder zwischen 200 Mikrometer und 2000 Mikrometer oder zwischen 300 Mikrometer und 2000 Mikrometer oder mehr als 250 Mikrometer bis zu 2000 Mikrometer oder mehr als 500 Mikrometer bis zu 2000 Mikrometer umfasst und/oder einen Schritt des Bereitstellens eines bestimmten Feuchtigkeitsgehalts, in Form von Wasser oder einer wässrigen Lösung oder einer Salzlösung, und des Hinzufügens des besagten Feuchtigkeitsgehalts zu der besagten zweiten keramischen Komponente, um eine zweite feuchte keramische Komponente zu erhalten, und des Hinzufügens der besagten zweiten feuchten keramischen Komponente zu dem besagten Pulveranteil der besagten ersten Strukturkomponente, vor dem besagten Schritt des Hinzufügens des besagten Flüssigkeitsanteils der besagten ersten Strukturkomponente, umfasst.

14. Verfahren nach einem oder mehreren der Ansprüche 10 bis 13, umfassend einen Schritt des Hinzufügens mindestens eines röntgendichten Materials und/oder einer pharmazeutischen oder medizinischen Substanz, wie beispielsweise mindestens eines Antibiotikums, zu der besagten ersten Strukturkomponente und/oder zu dem besagten Pulveranteil der besagten ersten Strukturkomponente und/oder dem besagten Flüssigkeitsanteil der besagten ersten Strukturkomponente und/oder der besagten zweiten keramischen Komponente und/oder einen Schritt des Eintauchens des besagten Biomaterials in eine Lösung, umfassend mindestens eine pharmazeutische oder medizinische Substanz.

15. Kit zum Füllen von Läsionen, Lücken oder Knochenkavitäten oder zur funktionellen Wiederherstellung von Geweben, worin die Ersetzung oder die Wiederherstellung von Knochengewebe im Gebrauch notwendig ist, umfassend ein Biomaterial in Form eines vorgeformten Feststoffs nach irgendeinem der Ansprüche 1 bis 9 und ein Biomaterial in flüssiger Form, worin das besagte Biomaterial in flüssiger Form eine erste Strukturkomponente in verfestigbarer flüssiger Form und eine zweite keramische Komponente umfasst, worin die besagte erste Strukturkomponente in flüssiger Form der ersten Strukturkomponente des besagten Biomaterials in Form eines vorgeformten Feststoffs entspricht oder davon verschieden ist und die besagte zweite keramische Komponente des besagten Biomaterials in flüssiger Form der besagten zweiten keramischen Komponente des besagten Biomaterials in Form eines vorgeformten Feststoffs entspricht oder davon verschieden ist.

16. Kit nach dem vorangegangenen Anspruch, worin das besagte Biomaterial in flüssiger Form im Gebrauch als ein Klebstoff und Haftmittel an der Implantationsstelle für das besagte Biomaterial in Form eines vorgeformten Feststoffs wirkt.

17. Biomaterial nach irgendeinem der Ansprüche 1 bis 9 oder Kit nach Anspruch 15 oder 16, zum Gebrauch als ein Knochenersatz zum Füllen von Läsionen, Lücken oder Knochenkavitäten oder zur funktionellen Wiederherstellung von Geweben, worin die Ersetzung oder die Wiederherstellung von Knochengewebe im Gebrauch notwendig sind, zum Beispiel bei großen Knochenlücken oder -kavitäten, großen Läsionen infolge von Kriegsverletzungen, großen Tumoren, schweren Traumata usw.

18. Knochenersatz zum Füllen von Knochenläsionen, Lücken oder Kavitäten oder zur funktionellen Wiederherstellung von Geweben, in denen die Ersetzung oder die Wiederherstellung von Knochengewebe im Gebrauch notwendig ist, zum Beispiel bei großen Knochenlücken oder -kavitäten, großen Läsionen infolge von Kriegsverletzungen, großen Tumoren, schweren Traumata usw., umfassend ein Biomaterial-Komposit nach irgendeinem der Ansprüche 1 bis 9, umfassend eine erste Strukturkomponente, eines permanenten und nicht-resorbierbaren Typs im Gebrauch im menschlichen Körper, und eine zweite keramische Komponente, eines löslichen oder solubilisierbaren Typs, worin die besagte erste Strukturkomponente ein organisches, Kunststoff- und/oder polymeres Material umfasst, worin die besagte erste Strukturkomponente eine miteinander verbundene Porosität enthält, umfassend Poren und/oder Kanälchen mit einem Durchmesser von weniger als 100 Mikrometer, Mikrokavitäten mit einer Größe von weniger als 100 Mikrometer und Makrokavitäten, worin mindestens einige der besagten Mikrokavitäten und der besagten Makrokavitäten im Gebrauch zur Aufnahme der besagten zweiten keramischen Komponente geeignet sind, worin die besagte zweite keramische Komponente mindestens ein Calciumsalz umfasst und in Form eines Pulvers mit einer Größe von weniger als 100 Mikrometer sowie von Granulaten mit einer Größe zwischen 100 Mikrometer und 2000 Mikrometer vorliegt, worin die besagte Porosität homogen über das gesamte Volumen, das von dem besagten Knochenersatz eingenommen wird, verteilt ist, **dadurch gekennzeichnet, dass** das besagte Biomaterial in Form eines vorgeformten Feststoffs vorliegt und eine Druckfestigkeit von mehr als 55 MPa aufweist, gemessen gemäß der Norm ISO 5833, zweite Ausgabe vom 01.05.2002.

19. Knochenersatz nach Anspruch 18, worin die besagten Mikrokavitäten und die besagten Makrokavitäten im Gebrauch hohl ausgebildet sind durch die Solubilisierung der besagten zweiten keramischen Komponente, des löslichen oder solubilisierbaren Typs im Gebrauch in Kontakt mit Wasser, einer Flüssigkeit, zum Beispiel einer biologischen Flüssigkeit, worin die besagte zweite keramische Komponente mindestens ein Calciumsalz umfasst.

20. Knochenersatz nach Anspruch 18 oder 19, worin die besagten Makrokavitäten eine Größe zwischen 100 und 500 Mikrometer oder zwischen 200 Mikrometer und 500 Mikrometer oder zwischen 300 Mikrometer und 500 Mikrometer oder zwischen 100 Mikrometer und 2000 Mikrometer oder zwischen 200 Mikrometer und 2000 Mikrometer oder zwischen 300 Mikrometer und 2000 Mikrometer oder mehr als 250 Mikrometer bis zu 2000 Mikrometer oder mehr als 500 Mikrometer bis zu 2000 Mikrometer aufweisen.

21. Knochenersatz nach irgendeinem der Ansprüche 18 bis 20, worin die besagte erste Strukturkomponente eine Oberfläche umfasst, die dazu ausgelegt ist, im Gebrauch an der Implantationsstelle mit dem Knochengewebe in Kontakt zu kommen, worin die besagte Oberfläche eine äußere Oberfläche des besagten Knochenersatzes und eine innere Oberfläche umfasst, die durch die Wände gegeben ist, die die besagte Porosität bilden und/oder umgeben, worin die besagte Oberfläche faltig und/oder strukturiert ist.

## Revendications

1. Biomatériau composite, biocompatible, ostéoinducteur et/ou ostéoconducteur, apte à être utilisé pendant l'utilisation comme un substitut osseux pour combler des lésions, des espaces ou des cavités osseuses, ou pour la restauration fonctionnelle des tissus nécessitant le remplacement ou la restauration de tissu osseux, comportant un premier composant structurel, d'un type permanent et non résorbable pendant l'utilisation dans le corps humain, dans lequel ledit premier composant structurel comporte un matériau organique, plastique et/ou polymère, et un deuxième composant céramique, d'un type soluble ou solubilisable pendant l'utilisation en contact avec un liquide, par exemple un liquide biologique, dans lequel ledit premier composant structurel comporte une porosité interconnectée comportant des pores et/ou des canalicules d'un diamètre inférieur à 100 microns, dans lequel ledit deuxième composant céramique, logé dans ladite porosité, comprend au moins un sel de calcium, dans lequel ledit deuxième composant céramique est sous la forme d'une poudre d'une taille inférieure à 100 microns et de granules d'une taille comprise entre 100 microns et 2000 microns, dans lequel ledit biomatériau composite est obtenu par le procédé de l'une quelconque des revendications 10 à 14, **caractérisé en ce que** ledit biomatériau est sous la forme d'un solide préformé et dans lequel ledit biomatériau présente une résistance à la compression supérieure à 55 MPa, mesurée conformément à la norme ISO5833, deuxième édition du 01-05-2002.

2. Biomatériau selon la revendication 1, dans lequel ledit biomatériau est compact et/ou présente une conformation tridimensionnelle sphérique, hémisphérique, polyédrique, parallélépipédique, hexaédrique, en cône tronqué ou en pyramide tronquée, en cône ou en pyramide, paralléliforme, en forme de cale, en forme de coin ou une forme tridimensionnelle irrégulière.

3. Biomatériau selon la revendication 1 ou 2, présentant une résistance à la compression supérieure à 60 MPa, ou supérieure à 70 MPa ou supérieure à 67-75 MPa, mesurée conformément à la norme ISO5833, deuxième édition du 01-05-2002.

4. Biomatériau selon quelconque des revendications précédentes, dans lequel lesdits granules présentent une taille comprise entre 100 microns et 500 microns ou entre 200 microns et 500 microns ou entre 200 microns et 2000 microns et/ou dans lequel ledit sel de calcium dudit deuxième composant céramique comporte un composé à base de phosphate de calcium (CP), un sulfate de calcium (CS), carbonate de calcium (CC), dans lequel ledit composé de phosphate de calcium comporte au moins un ou plusieurs de : dihydrogénophosphate de calcium monohydraté (MCPM), dihydrogénophosphate de calcium anhydre (MCPA), monohydrogénophosphate de calcium dihydraté (DCPD), monohydrogénophosphate de calcium anhydre (DCP), phosphate octacalcique (OCP), phosphate alpha tricalcique (α-TCP), phosphate bêta-tricalcique (β-TCP), hydroxyapatite (HA), phosphate tétracalcique (TTCP).

5. Biomatériau selon quelconque des revendications précédentes, dans lequel ledit deuxième composant céramique est réparti de manière homogène dans tout le volume occupé par ledit biomatériau, au moins pendant une première période pendant l'utilisation après l'implantation.

6. Biomatériau selon la revendication précédente, capable, après ladite première période pendant l'utilisation après l'implantation, de ne plus comporter ledit deuxième composant céramique, puisque ledit deuxième composant céramique pendant l'utilisation s'est dissous et/ou solubilisé.

7. Biomatériau selon quelconque des revendications précédentes, dans lequel ledit premier composant structurel comporte une porosité comportant en outre des microcavités d'une taille inférieure à 100 microns et des macrocavités d'une taille comprise entre 100 et 500 microns ou entre 200 microns et 500 microns ou entre 300 microns et 500 microns ou entre 100 microns et 2000 microns ou entre 200 microns et 2000 microns ou entre 300 microns et 2000 microns ou supérieure à 250 microns et jusqu'à 2000 microns ou supérieure à 500 microns et jusqu'à 2000 microns, dans laquel ladite porosité étant interconnectée et répartie de manière homogène dans tout le volume occupé par ledit biomatériau et/ou dans lequel ledit deuxième composant céramique est logé, pendant l'utilisation au moins pendant ladite première période pendant l'utilisation après l'implantation, à l'intérieur de ladite porosité formée par lesdites microcavités et lesdites macrocavités dudit premier composant structurel.

8. Biomatériau selon quelconque des revendications précédentes, dans lequel ledit matériau organique, plastique et/ou polymère dudit premier composant structurel comporte ou est constitué d'un matériau plastique, une résine plastique, acrylique ou méthacrylique, ou un polymère plastique ou acrylique ou un ciment osseux en polyméthacrylate de méthyle (PMMA) et/ou un copolymère à base de méthacrylate de méthyle ou un polymère thermoplastique, polyéthylène, polypropylène, polyester, un polymère thermoformable ou d'autres matériaux similaires.

9. Biomatériau selon quelconque des revendications précédentes, comportant un niveau d'humidité, sous forme d'eau ou de solution aqueuse ou de solution saline et/ou au moins un matériau radio-opaque, et/ou une substance pharmaceutique ou médicale, telle qu'au moins un antibiotique.

10. Procédé pour fabriquer un biomatériau composite selon l'une ou plusieurs des revendications précédentes, dans lequel ledit biomatériau composite est biocompatible, ostéoinducteur et/ou ostéoconducteur, apte à être utilisé pendant l'utilisation comme un substitut osseux pour combler des lésions, des espaces ou des cavités osseuses ou pour la restauration fonctionnelle de tissus nécessitant le remplacement ou la restauration de tissu osseux pendant l'utilisation, comportant un premier composant structurel, d'un type permanent et non résorbable utilisé dans le corps humain, et un deuxième composant céramique d'un type soluble ou solubilisable pendant l'utilisation au contact d'un liquide, par exemple un liquide biologique, comportant les étapes suivantes :
fournir ledit premier composant structurel comportant un matériau organique, plastique et/ou polymère, dans lequel ledit premier composant comporte une partie en poudre et une partie liquide, dans lequel ladite partie en poudre comporte une partie en poudre polymère, capable de réagir avec ladite partie liquide,
fournir ledit deuxième composant céramique comportant au moins un sel de calcium, dans lequel ledit deuxième composant céramique est sous la forme d'une poudre d'une taille inférieure à 100 microns et de granules d'une taille comprise entre 100 microns et 2000 microns,
mélanger ledit deuxième composant céramique avec ladite partie en poudre dudit premier composant céramique, afin d'obtenir un mélange de particules avec distribution homogène des deux composants,
ajouter ladite partie liquide dudit premier composant structurel audit mélange de particules,
mélanger ladite partie liquide et ledit mélange de particules de sorte que ladite partie liquide réagisse avec ladite partie en poudre dudit premier composant structurel pour former un biomatériau sous forme fluide dans lequel ledit deuxième composant céramique est réparti de manière homogène,
polymériser et/ou solidifier, par exemple dans un moule spécial et/ou dans un four et/ou dans un autoclave, ledit biomatériau sous forme fluide, jusqu'à ce qu'il se solidifie,
soumettre ledit biomatériau sous forme fluide à une pression positive comprise entre +4 et +12 bars, obtenant ainsi une compaction du biomatériau et une réduction du volume et/ou de la taille de toutes les bulles d'air et/ou d'eau ou d'humidité présentes et éventuellement piégées dans le biomatériau pendant ladite étape de polymérisation et/ou de solidification,
obtenir ledit biomatériau sous la forme d'un solide préformé, ayant une résistance à la compression supérieure à 55 MPa, mesurée conformément à la norme ISO5833, deuxième édition du 01-05-2002.

11. Procédé selon la revendication 10, dans lequel ladite étape de soumission à une pression positive a lieu pendant ladite étape de polymérisation et/ou de solidification.

12. Procédé selon la revendication 10 ou 11, dans lequel ladite étape de soumission comporte en outre la soumission dudit biomatériau sous forme fluide à une température élevée, inférieure à 100°C.

13. Procédé selon l'une ou plusieurs des revendications 10 à 12, dans lequel ladite étape de fourniture dudit deuxième composant céramique comporte la fourniture dudit deuxième composant céramique sous forme de granulés d'une taille comprise entre 100 microns et 500 microns ou entre 200 microns et 500 microns ou entre 300 et 500 microns ou entre 200 microns et 2000 microns ou entre 300 microns et 2000 microns ou supérieure à 250 microns jusqu'à 2000 microns ou supérieure à 500 microns jusqu'à 2000 microns et/ou comportant une étape de fourniture d'un certain niveau d'humidité, sous forme d'eau ou de solution aqueuse ou de solution saline, et d'ajout dudit niveau d'humidité audit deuxième composant céramique, obtenant un deuxième composant céramique humide et ajoutant ledit deuxième composant céramique humide à ladite partie en poudre dudit premier composant structurel, avant ladite étape d'ajout de ladite partie liquide dudit premier composant structurel.

14. Procédé selon l'une ou plusieurs des revendications 10 à 13, comportant une étape d'ajout d'au moins un matériau radio-opaque et/ou d'une substance pharmaceutique ou médicale, telle qu'au moins un antibiotique, audit premier composant structurel et/ou à ladite partie en poudre dudit premier composant structurel et/ou à ladite partie liquide dudit premier composant structurel et/ou audit deuxième composant céramique et/ou une étape d'immersion dudit biomatériau dans une solution comportant au moins une substance pharmaceutique ou médicale.

15. Kit pour combler des lésions, des espaces ou des cavités osseuses ou pour la restauration fonctionnelle de tissus nécessitant le remplacement ou la restauration de tissu osseux pendant l'utilisation, comportant un biomatériau sous la forme d'un solide préformé selon quelconque des revendications 1 à 9 et un biomatériau sous forme fluide, dans lequel ledit biomatériau sous forme fluide comporte un premier composant structurel sous forme fluide solidifiable et un deuxième composant céramique, dans lequel ledit premier composant structurel sous forme fluide est identique ou différent du premier composant structurel dudit biomatériau sous la forme d'un solide préformé et ledit deuxième composant céramique dudit biomatériau sous forme fluide est identique ou différent dudit deuxième composant céramique dudit biomatériau sous forme de solide préformé.

16. Kit selon la revendication précédente, dans lequel ledit biomatériau sous forme fluide agit comme une colle et adhésif pendant l'utilisation sur le site d'implantation dudit biomatériau sous la forme d'un solide préformé.

17. Biomatériau selon l'une quelconque des revendications 1 à 9 ou kit selon la revendication 15 ou 16, destiné à être utilisé comme un substitut osseux pour combler des lésions, des espaces ou des cavités osseuses ou pour la restauration fonctionnelle de tissus dans lesquels le remplacement ou la restauration de tissu osseux est nécessaire pendant l'utilisation, par exemple pour les grands espaces ou cavités osseuses, les grandes lésions dues à des blessures de guerre, les grandes tumeurs, les traumatismes majeurs, etc.

18. Substitut osseux pour combler les lésions, les espaces ou les cavités osseuses ou pour la restauration fonctionnelle des tissus lorsque le remplacement ou la restauration de tissu osseux est nécessaire pendant l'utilisation, par exemple pour les grands espaces ou cavités osseuses, les grandes lésions dues à des blessures de guerre, les grands tumeurs, les traumatismes majeurs, etc., comportant un biomatériau composite selon l'une quelconque des revendications 1 à 9, composant un premier composant structurel, d'un type permanent et non résorbable pendant l'utilisation dans le corps humain, et un deuxième composant céramique, du type soluble ou solubilisable, dans lequel le premier composant structurel comporte un matériau organique, plastique et/ou polymère, dans lequel ledit premier composant structurel comprend une porosité interconnectée comportant des pores et/ou des canalicules de diamètre inférieur à 100 microns, des microcavités de taille inférieure à 100 microns, et des macrocavités, dans lequel au moins certaines desdites microcavités et lesdites macrocavités sont apte à loger pendant l'utilisation ledit deuxième composant céramique, dans lequel ledit deuxième composant céramique comporte au moins un sel de calcium et est sous la forme d'une poudre d'une taille inférieure à 100 microns et de granules d'une taille comprise entre 100 microns et 2000 microns, dans lequel ladite porosité est répartie de manière homogène dans tout le volume occupé par ledit substitut osseux, **caractérisé en ce que** ledit biomatériau est sous la forme d'un solide préformé et présente une résistance à la compression supérieure à 55 MPa, mesurée conformément à la norme ISO5833, deuxième édition du 01-05-2002.

19. Substitut osseux selon la revendication 18, dans lequel lesdites microcavités et lesdites macrocavités sont rendues creuses pendant l'utilisation par la solubilisation dudit deuxième composant céramique, de type soluble ou solubilisable pendant l'utilisation au contact de l'eau, d'un liquide, par exemple un liquide biologique, dans lequel ledit deuxième composant céramique comporte au moins un sel de calcium.

20. Substitut osseux selon la revendication 18 ou 19, dans lequel lesdites macrocavités présentent une taille comprise entre 100 et 500 microns ou entre 200 microns et 500 microns ou entre 300 microns et 500 microns ou entre 100 microns et 2000 microns ou entre 200 microns et 2000 microns ou entre 300 microns et 2000 microns ou supérieure à 250 microns jusqu'à 2000 microns ou supérieure à 500 microns jusqu'à 2000 microns.

21. Substitut osseux selon l'une quelconque des revendications 18 à 20, dans lequel ledit premier composant structurel comporte une surface adaptée pour entrer en contact avec le tissu osseux pendant l'utilisation au niveau du site d'implantation, dans lequel ladite surface comporte une surface externe dudit substitut osseux et une surface interne, donnée par les parois qui constituent et/ou entourent ladite porosité, dans lequel ladite surface est plissée et/ou texturée.
